# EUROPEAN PATENT SPECIFICATION

(11) **EP 4 017 512 B1**
(45) Date of publication and mention of the grant of the patent: **02.04.2025**
(21) Application number: 20776222.0
(22) Date of filing: 20.08.2020
(51) Int. Cl.: A61K 35/28, C12N 5/00, A61P 29/00, A61P 37/02

(54) **COMPOSITIONS FOR MONOCYTE AND MACROPHAGE POLARIZATION AND METHODS OF USE**
ZUSAMMENSETZUNGEN FÜR MONOZYTEN- UND MAKROPHAGENPOLARISATION UND VERWENDUNGSVERFAHREN
COMPOSITIONS POUR LA POLARISATION DE MONOCYTES ET DE MACROPHAGES ET MÉTHODES D'UTILISATION

(30) Priority: 23.08.2019 US 201962891188 P
(43) Date of publication of application: 29.06.2022
(73) Proprietor: Orbsen Therapeutics Limited, Dangan H91 A3EF Galway (IE)
(72) Inventor: ELLIMAN, Stephen, J., Galway Business Park Dangan Galway H91 A3EF (IE); ALAGESAN, Senthilkumar, Galway Business Park Dangan Galway H91 A3EF (IE); DEEDIGAN, Laura, M., Galway Business Park Dangan Galway H91 A3EF (IE)
(74) Representative: Schlich
(86) International application number: PCT/IB2020/000680
(87) International publication number: WO 2021/038289

(56) References cited:
- WO-A1-2010/119039
- WO-A1-2017/122095
- WO-A2-2011/160055
- WO-A2-2016/026854
- WO-A2-2019/012334

## Description

### BACKGROUND

Mitochondrial function has been recognized to decline during aging concomitant with mitochondrial morphological changes such as abnormally rounded mitochondria and reduced mitochondrial numbers and decreases in mitochondrial DNA. Further, it is recognized that mitochondrial dysfunction plays a role in onset of various diseases. Restoration of mitochondrial numbers and function has the potential to treat these diseases and reverse some of the effects of aging on cellular function and metabolism.

### SUMMARY

The present invention provides a population of SDC2+ stromal stem cells and a method for preparing such a population of cells, as defined in the claims.
Described herein are methods of treating an inflammatory disease in an individual. The methods comprise: administering a population of stromal stem cells to the individual in an amount effective to reduce at least one symptom of the inflammatory disease, wherein the population of mammalian stromal stem cells has been treated with an agent comprising: i) an agent that reduces expression of CDC50A; ii) an agent that increases expression of IRG1/ACOD1; iii) an agent that increases expression of PGC1α; or iv) an agent that reduces expression of CD47. In some embodiments, the population of mammalian stromal stem cells has reduced CDC50 activity. In some embodiments, the population of mammalian stromal stem cells has increased IRG1/ACOD1 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. Alternatively and/or additionally, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also provided herein are methods of treating an individual, and the methods comprise administering to the individual a population of mammalian stromal stem cells, wherein the population of mammalian stromal stem cells has reduced CDC50 activity. In some embodiments, the population of mammalian stromal stem cells has reduced CDC50 activity. In some embodiments, the population of mammalian stromal stem cells has increased IRG1/ACOD1 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also described herein are methods of treating an individual, and the methods comprise administering to the individual a population of mammalian stromal stem cells, wherein the population of mammalian stromal stem cells has increased IRG1/ACOD1 activity. In some embodiments, the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also described herein are methods of treating an individual, and the methods comprise administering to the individual a population of mammalian stromal stem cells, wherein the population of mammalian stromal stem cells has increased expression of PGC1α. In some embodiments, the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also described herein are methods of treating an individual, and the methods comprise administering to the individual a population of mammalian stromal stem cells, wherein the population of mammalian stromal stem cells has reduced expression of CD47. In some embodiments, the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also described herein are methods of treating an individual, and methods comprises administering to the individual a population of mammalian stromal stem cells, wherein the population of mammalian stromal stem cells has been treated with an agent comprising: i) an agent that reduces expression of CDC50A; ii) an agent that increases expression of IRG1/ACOD1; iii) an agent that increases expression of PGC1α; or iv) an agent that reduces expression of CD47; and wherein at least 30% of the population of mammalian stromal stem cells is positive for SDC2. It is preferred that the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also described herein are methods of preparing a population of stromal stem cells, and the methods comprise a) obtaining a population of mammalian cells; b) treating the population of mammalian cells with at least one of: i) an agent that reduces expression of CDC50A; ii) an agent that increases expression of IRG1/ACOD1; iii) an agent that increases expression of PGC1α; and iv) an agent that reduces expression of CD47; and c) isolating SDC2+ cells from the treated population of mammalian cells. In some embodiments, the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. In some embodiments, the stromal stem cell is a human cell, a mouse cell, a rat cell, or an equine cell. In some embodiments, the population of mammalian cells is obtained from a source selected from bone marrow, umbilical cord, umbilical cord blood, adipose tissue, skeletal muscle, endometrium, placenta, Wharton's jelly, and cells derived from pluripotent cells. In some embodiments, the population of mammalian cells is exposed to a low oxygen environment. In some embodiments, isolating SDC2+ cells from the treated population of mammalian cells comprises contacting the treated population of mammalian cells to an SDC2 binding agent and isolating cells bound to the SDC2 binding agent. In such embodiments, the SDC2 binding agent may comprise an anti-SDC2 antibody or fragment thereof. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell.

Also provided herein are methods of treating an individual, and methods comprise administering to the individual a composition comprising exosomes isolated from a population of mammalian stromal stem cells, wherein the population of mammalian stromal stem cells has reduced expression of CDC50. In some embodiments, the population of mammalian stromal stem cells has increased IRG1/ACOD1 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also described herein are methods of treating an individual, and methods comprise administering to the individual a composition comprising exosomes isolated from a population of mammalian stromal stem cells, wherein the population of mammalian stromal stem cells has increased expression of IRG1/ACOD1. In some embodiments, the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also described herein are methods of treating an individual, and methods comprise administering to the individual a composition comprising exosomes isolated from a population of mammalian stromal stem cells, wherein the population of mammalian stromal stem cells has increased expression of PGC1α. In some embodiments, the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also described herein are methods of treating an individual, and methods comprise administering to the individual a composition comprising exosomes isolated from a population of mammalian stromal stem cells, wherein the population of mammalian stromal stem cells has reduced expression of CD47. In some embodiments, the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also described herein are methods of treating an individual, and methods comprise administering to the individual a composition comprising exosomes isolated from a population of mammalian stromal stem cells, wherein the population of mammalian stromal stem cells has been treated with an agent comprising: i) an agent that reduces expression of CDC50A; ii) an agent that increases expression of IRG1/ACOD1; iii) an agent that increases expression of PGC1α; or iv) an agent that reduces expression of CD47; and wherein at least 30% of the population of mammalian stromal stem cells is positive for SDC2. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell. In some embodiments, the individual exhibits at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

Also described herein are methods of preparing a composition comprising exosomes isolated from a population of stromal stem cells, and methods comprise a) obtaining a population of mammalian cells; b) treating the population of mammalian cells with at least one of: i) an agent that reduces expression of CDC50A; ii) an agent that increases expression of IRG1/ACOD1; iii) an agent that increases expression of PGC1α; and iv) an agent that reduces expression of CD47; and c) isolating SDC2+ exosomes from the treated population of mammalian cells. In some embodiments, the population of mammalian stromal stem cells has reduced CDC50 activity. In some embodiments, the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. In some embodiments, the stromal stem cell is a human cell, a mouse cell, a rat cell, or an equine cell. In some embodiments, the population of mammalian cells is obtained from a source selected from bone marrow, umbilical cord, umbilical cord blood, adipose tissue, skeletal muscle, endometrium, placenta, Wharton's jelly, and cells derived from pluripotent cells. In some embodiments, the population of mammalian cells is exposed to a low oxygen environment. In some embodiments, isolating SDC2+ cells from the treated population of mammalian cells comprises contacting the treated population of mammalian cells to an SDC2 binding agent and isolating cells bound to the SDC2 binding agent. In such embodiments, the SDC2 binding agent may comprise an anti-SDC2 antibody or fragment thereof. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell.

Also provided herein are methods of measuring a potency of a batch of stromal stem cells, the methods comprise(a) isolating a population of SDC2+ stromal stem cells from a mixed population of mammalian cells; and (b) measuring at least one of (i) an expression level of IRG1/ACOD1 and (ii) a release level of itaconate. In some embodiments, the expression level of IRG1/ACOD1 is measured by QPCR or quantitative FACS. In some embodiments, the release level of itaconate is measured by mass spectrometry. In some embodiments, the mixed population of mammalian cells is bone marrow cells or umbilical cord cells. In some embodiments, the mixed population of mammalian cells is human. In some embodiments, the method further comprises treating the population of mammalian cells with at least one of: i) an agent that reduces expression of CDC50A; ii) an agent that increases expression of IRG1/ACOD1; iii) an agent that increases expression of PGC1α; and iv) an agent that reduces expression of CD47. In such embodiments, it is contemplated that wherein the population of mammalian stromal stem cells has reduced CDC50 activity. In some embodiments, the population of mammalian stromal stem cells has increased PGC1α activity. In some embodiments, the population of mammalian stromal stem cells has reduced CD47 activity. In some embodiments, the population of mammalian stromal stem cells is at least 30% positive for SDC2. In some embodiments, CDC50 activity is reduced by treating the population of mammalian stromal stem cells with at least one of a CDC50 antagonist, a CDC50 inhibitory nucleic acid, and a CDC50 inhibitory peptide. In some embodiments, IRG1/ACOD1 activity is increased by treating the population of mammalian stromal stem cells with at least one of an IRG1/ACOD1 agonist and a nucleic acid encoding IRG1/ACOD1. In some embodiments, PGC1α activity is increased by treating the population of mammalian stromal stem cells with at least one of a PGC1α agonist and a nucleic acid encoding PGC1α. In some embodiments, CD47 activity is reduced by treating the population of mammalian stromal stem cells with at least one of a CD47 antagonist, a CD47 inhibitory nucleic acid, and a CD47 inhibitory peptide. In some embodiments, the stromal stem cell is a human cell, a mouse cell, a rat cell, or an equine cell. In some embodiments, the population of mammalian cells is obtained from a source selected from bone marrow, umbilical cord, umbilical cord blood, adipose tissue, skeletal muscle, endometrium, placenta, Wharton's jelly, and cells derived from pluripotent cells. In some embodiments, the population of mammalian cells is exposed to a low oxygen environment. In some embodiments, isolating SDC2+ cells from the treated population of mammalian cells comprises contacting the treated population of mammalian cells to an SDC2 binding agent and isolating cells bound to the SDC2 binding agent. In such embodiments, the SDC2 binding agent may comprise an anti-SDC2 antibody or fragment thereof. It is preferred that in some embodiments, the method increases the total number of mitochondria in the cell, and/or the method increases the total volume of mitochondria in the cell, and/or the method increases phosphatidylserine (PS) levels on the cell surface, and/or the method increases itaconate levels in the cell.

### BRIEF DESCRIPTION OF THE DRAWINGS

An understanding of the features and advantages of the present invention will be obtained by reference to the following detailed description that sets forth illustrative embodiments, in which the principles of the invention are utilized, and the accompanying drawings of which:
**Figs. 1A-D** show correlation between the amount of indoleamine 2,3-dioxygenase (IDO) produced and the T cell inhibitory potential of MSC donors.
**Figs. 2A-D** show that IDO-1^{hi} DP donor is capable of inducing immune regulatory monocytes in comparison to IDO-1^{lo}DP donor in a 24hr monocyte assay.
**Figs. 3A-C** show that reduction in TMEM30A expression results in significantly increased PtS on the cell surface of ORBCEL-C.
**Figs. 4A-C** show impact of siTMEM30A on ORBCEL-C phenotype.
**Figs. 5A-B** show viability and morphology of siTMEM30A-treated ORBCEL-C.
**Figs. 6A-D**show that reduction in TMEM30A expression and increased PtS results in higher IDO-1 expression (converts IDO-1^{lo} to IDO-1^{hi}
**Figs. 7A-C** show that TMEM30A KD Switches IDO-1^{lo} donor's M2 monocytes induction from Low to High (CA49DP = IDO-1^{hi} & CA58DP = IDO-1^{lo}).
**Figs. 8A-C** show that induction of regulatory monocytes (CD163⁺ CD206⁺) by IDO-1^{hi} DP is via an active cell-cell interaction process.
**Figs. 9A-C** show that IDO-1^{hi} donor primed monocytes are capable of inducing regulatory T-cells (T-regs).

### DETAILED DESCRIPTION

Stromal stem cells have shown utility in methods of treatment for a variety of diseases spanning organ systems. In particular, stromal stem cells and exosomes isolated from stromal stem cells show promise in treating inflammatory disease such as inflammatory liver disease, inflammatory kidney disease, inflammatory lung disease, and skin ulcers. In particular, stromal stem cell populations having at least 30% SDC2+ cells and exosomes isolated from these cells are thought to be useful therapeutics for disease.

A mechanism of action for SDC2+ stromal stem cell and exosome compositions has long eluded researchers. Disclosed herein is the discovery that the therapeutic effect of stromal stem cells and exosomes isolated from stromal stem cells is in delivery of mitochondria to diseased cells and tissue. Further, it has been found that stromal stem cells improve monocyte and macrophage polarization to induce an M2 phenotype. Accordingly, SDC2+ stromal stem cells and exosomes therefrom treated to have increased capability of mitochondrial transfer have improved therapeutic efficacy over untreated SDC2+ stromal stem cells.

### Compositions for Monocyte and Macrophage Polarization

Provided herein are compositions comprising stromal stem cells and exosome compositions having increased capabilities of monocyte and macrophage polarization to M2 phenotype, such as SDC2+ stromal stem cells and SDC2+ exosomes treated to have at least one of increased mitochondrial numbers and increased mitochondrial density compared to untreated SDC2+ stromal stem cells and SDC2+ exosomes. Additionally, stromal stem cell and exosome compositions herein are treated to increase cell surface phosphatidylserine (PS). Further, stromal stem cell and exosome compositions herein are treated to have increased itaconate in the cells.

Compositions for increasing monocyte and macrophage polarization to M2 phenotype comprising stromal stem cells and exosomes, including SDC2+ stromal stem cells and SDC2+ exosomes, are treated to increase their capacity for monocyte and macrophage polarization to M2 phenotype. Non-limiting examples of modifying SDC2+ stromal stem cells and SDC2+ exosomes include but are not limited to decreasing expression of CDC50, inhibiting CDC50 activity, increasing expression of IRG1/ACOD1, increasing IRG1/ACOD1 activity, decreasing expression of CD47, inhibiting CD47, increasing expression of PGC1α, and increasing PGC1α activity, including combinations of the above modifications. In some cases, stromal stem cells are isolated from bone marrow or umbilical cord tissue. In some cases, compositions for mitochondrial transfer comprising SDC2+ stromal stem cells and SDC2+ exosomes are treated to decrease expression of CDC50 or treated to inhibit CDC50 activity. Alternatively or in combination, compositions for mitochondrial transfer comprising SDC2+ stromal stem cells and SDC2+ exosomes are treated to increase expression of IRG1/ACOD1 or treated to increase IRG1/ACOD1 activity. Alternatively or in combination, compositions for mitochondrial transfer comprising SDC2+ stromal stem cells and SDC2+ exosomes are treated to decrease expression of CD47 or treated to inhibit CD47 activity. Alternatively or in combination, compositions for mitochondrial transfer comprising SDC2+ stromal stem cells and SDC2+ exosomes are treated to increase expression of PGC1α or treated to increase PGC1α activity. Exemplary treatments for decreasing expression of CDC50 and/or CD47 include but are not limited to gene knockout and RNAi methods, using CRISPR or siRNA techniques disclosed elsewhere herein. Exemplary treatments for increasing expression of PGC1α and/or IRG1/ACOD1 include but are not limited to transfecting or transducing the cells with a plasmid or a virus that overexpresses PGC1α and/or IRG1/ACOD1.

In certain aspects, provided herein are compositions for increasing monocyte and macrophage polarization to M2 phenotype comprising a stromal stem cell treated to decrease expression of or inhibit CDC50. In some cases, the stromal stem cell is an SDC2+ stromal stem cell. In some cases, the stromal stem cell is treated with a CDC50 siRNA. In some cases, the stromal stem cell is treated with a virus expressing a shRNA targeting CDC50. In some cases, the stromal stem cell is treated with a CDC50 inhibitor. In some cases, CDC50 expression is reduced by 10% in a treated stromal stem cell compared to an untreated stromal stem cell. In some cases, CDC50 expression or activity is reduced by 20% in a treated stromal stem cell compared to an untreated stromal stem cell. In some cases, CDC50 expression or activity is reduced by 30% in a treated stromal stem cell compared to an untreated stromal stem cell. In some cases, CDC50 expression or activity is reduced by 40% in a treated stromal stem cell compared to an untreated stromal stem cell. In some cases, CDC50 expression or activity is reduced by 50% in a treated stromal stem cell compared to an untreated stromal stem cell. In some cases, CDC50 expression or activity is reduced by 60% in a treated stromal stem cell compared to an untreated stromal stem cell. In some cases, CDC50 expression or activity is reduced by 70% in a treated stromal stem cell compared to an untreated stromal stem cell. In some cases, CDC50 expression or activity is reduced by 80% in a treated stromal stem cell compared to an untreated stromal stem cell. In some cases, CDC50 expression or activity is reduced by 90% in a treated stromal stem cell compared to an untreated stromal stem cell. In some cases, CDC50 expression or activity is reduced by 95% in a treated stromal stem cell compared to an untreated stromal stem cell. In some cases, CDC50 expression or activity is reduced by 99% in a treated stromal stem cell compared to an untreated stromal stem cell. In some cases, CDC50 expression or activity is reduced by 100% in a treated stromal stem cell compared to an untreated stromal stem cell. In some cases, stromal stem cells are isolated from bone marrow or umbilical cord tissue.

In certain aspects, described herein are compositions for increasing monocyte and macrophage polarization to M2 phenotype comprising a stromal stem cell treated to increase expression of or activate IRG1/ACOD1. In some cases, the stromal stem cell is an SDC2+ stromal stem cell. In some cases, the stromal stem cell is treated with an IRG1/ACOD1 plasmid. In some cases, the stromal stem cell is treated with a virus expressing IRG1/ACOD1. In some cases, the stromal stem cell is treated with an IRG1/ACOD1 agonist. In some cases, IRG1/ACOD1 expression is increased by 20% in a treated stromal stem cell compared to an untreated stromal stem cell. In some cases, IRG1/ACOD1 expression or activity is increased by 50% in a treated stromal stem cell compared to an untreated stromal stem cell. In some cases, IRG1/ACOD1 expression or activity is increased by 80% in a treated stromal stem cell compared to an untreated stromal stem cell. In some cases, IRG1/ACOD1 expression or activity is increased by 100% in a treated stromal stem cell compared to an untreated stromal stem cell. In some cases, IRG1/ACOD1 expression or activity is increased by 150% in a treated stromal stem cell compared to an untreated stromal stem cell. In some cases, IRG1/ACOD1 expression or activity is increased by 200% in a treated stromal stem cell compared to an untreated stromal stem cell. In some cases, IRG1/ACOD1 expression or activity is increased by 250% in a treated stromal stem cell compared to an untreated stromal stem cell. In some cases, IRG1/ACOD1 expression or activity is increased by 300% in a treated stromal stem cell compared to an untreated stromal stem cell. In some cases, IRG1/ACOD1 expression or activity is increased by 350% in a treated stromal stem cell compared to an untreated stromal stem cell. In some cases, IRG1/ACOD1 expression or activity is increased by 400% in a treated stromal stem cell compared to an untreated stromal stem cell. In some cases, IRG1/ACOD1 expression or activity is increased by 450% in a treated stromal stem cell compared to an untreated stromal stem cell. In some cases, IRG1/ACOD1 expression or activity is increased by 500% in a treated stromal stem cell compared to an untreated stromal stem cell. In some cases, stromal stem cells are isolated from bone marrow or umbilical cord tissue.

In certain aspects, described herein are compositions for increasing monocyte and macrophage polarization to M2 phenotype comprising a stromal stem cell treated to decrease expression of or inhibit CD47. In some cases, the stromal stem cell is an SDC2+ stromal stem cell. In some cases, the stromal stem cell is treated with a CD47 siRNA. In some cases, the stromal stem cell is treated with a virus expressing an shRNA targeting CD47. In some cases, the stromal stem cell is treated with a CD47 inhibitor. In some cases, CD47 expression is reduced by 10% in a treated stromal stem cell compared to an untreated stromal stem cell. In some cases, CD47 expression or activity is reduced by 20% in a treated stromal stem cell compared to an untreated stromal stem cell. In some cases, CD47 expression or activity is reduced by 30% in a treated stromal stem cell compared to an untreated stromal stem cell. In some cases, CD47 expression or activity is reduced by 40% in a treated stromal stem cell compared to an untreated stromal stem cell. In some cases, CD47 expression or activity is reduced by 50% in a treated stromal stem cell compared to an untreated stromal stem cell. In some cases, CD47 expression or activity is reduced by 60% in a treated stromal stem cell compared to an untreated stromal stem cell. In some cases, CD47 expression or activity is reduced by 70% in a treated stromal stem cell compared to an untreated stromal stem cell. In some cases, CD47 expression or activity is reduced by 80% in a treated stromal stem cell compared to an untreated stromal stem cell. In some cases, CD47 expression or activity is reduced by 90% in a treated stromal stem cell compared to an untreated stromal stem cell. In some cases, CD47 expression or activity is reduced by 95% in a treated stromal stem cell compared to an untreated stromal stem cell. In some cases, CD47 expression or activity is reduced by 99% in a treated stromal stem cell compared to an untreated stromal stem cell. In some cases, CD47 expression or activity is reduced by 100% in a treated stromal stem cell compared to an untreated stromal stem cell. In some cases, stromal stem cells are isolated from bone marrow or umbilical cord tissue.

In certain aspects, described herein are compositions for increasing monocyte and macrophage polarization to M2 phenotype comprising a stromal stem cell treated to increase expression of or activate PGC1α. In some cases, the stromal stem cell is an SDC2+ stromal stem cell. In some cases, the stromal stem cell is treated with a PGC1α plasmid. In some cases, the stromal stem cell is treated with a virus expressing PGC1α. In some cases, the stromal stem cell is treated with a PGC1α agonist. In some cases, PGC1α expression is increased by 20% in a treated stromal stem cell compared to an untreated stromal stem cell. In some cases, PGC1α expression or activity is increased by 50% in a treated stromal stem cell compared to an untreated stromal stem cell. In some cases, PGC1α expression or activity is increased by 80% in a treated stromal stem cell compared to an untreated stromal stem cell. In some cases, PGC1α expression or activity is increased by 100% in a treated stromal stem cell compared to an untreated stromal stem cell. In some cases, PGC1α expression or activity is increased by 150% in a treated stromal stem cell compared to an untreated stromal stem cell. In some cases, PGC1α expression or activity is increased by 200% in a treated stromal stem cell compared to an untreated stromal stem cell. In some cases, PGC1α expression or activity is increased by 250% in a treated stromal stem cell compared to an untreated stromal stem cell. In some cases, PGC1α expression or activity is increased by 300% in a treated stromal stem cell compared to an untreated stromal stem cell. In some cases, PGC1α expression or activity is increased by 350% in a treated stromal stem cell compared to an untreated stromal stem cell. In some cases, PGC1α expression or activity is increased by 400% in a treated stromal stem cell compared to an untreated stromal stem cell. In some cases, PGC1α expression or activity is increased by 450% in a treated stromal stem cell compared to an untreated stromal stem cell. In some cases, PGC1α expression or activity is increased by 500% in a treated stromal stem cell compared to an untreated stromal stem cell. In some cases, stromal stem cells are isolated from bone marrow or umbilical cord tissue.

In certain aspects, provided herein are compositions for increasing monocyte and macrophage polarization to M2 phenotype comprising an exosome treated to decrease expression of or inhibit CDC50. In some cases, the exosome is an SDC2+ exosome. In some cases, the exosome is isolated from a stromal stem cell is treated with a CDC50 siRNA. In some cases, the exosome is isolated from a stromal stem cell is treated with a virus expressing an shRNA targeting CDC50. In some cases, the exosome is isolated from a stromal stem cell is treated with a CDC50 inhibitor. In some cases, CDC50 expression is reduced by 10% in a treated stromal stem cell compared to an untreated stromal stem cell. In some cases, CDC50 expression or activity is reduced by 20% in a treated stromal stem cell compared to an untreated stromal stem cell. In some cases, CDC50 expression or activity is reduced by 30% in a treated stromal stem cell compared to an untreated stromal stem cell. In some cases, CDC50 expression or activity is reduced by 40% in a treated stromal stem cell compared to an untreated stromal stem cell. In some cases, CDC50 expression or activity is reduced by 50% in a treated stromal stem cell compared to an untreated stromal stem cell. In some cases, CDC50 expression or activity is reduced by 60% in a treated stromal stem cell compared to an untreated stromal stem cell. In some cases, CDC50 expression or activity is reduced by 70% in a treated stromal stem cell compared to an untreated stromal stem cell. In some cases, CDC50 expression or activity is reduced by 80% in a treated stromal stem cell compared to an untreated stromal stem cell. In some cases, CDC50 expression or activity is reduced by 90% in a treated stromal stem cell compared to an untreated stromal stem cell. In some cases, CDC50 expression or activity is reduced by 95% in a treated stromal stem cell compared to an untreated stromal stem cell. In some cases, CDC50 expression or activity is reduced by 99% in a treated stromal stem cell compared to an untreated stromal stem cell. In some cases, CDC50 expression or activity is reduced by 100% in a treated stromal stem cell compared to an untreated stromal stem cell. In some cases, stromal stem cells are isolated from bone marrow or umbilical cord tissue.

In certain aspects, described herein are compositions for increasing monocyte and macrophage polarization to M2 phenotype comprising an exosome treated to increase expression of or activate IRG1/ACOD1. In some cases, the exosome is an SDC2+ exosome. In some cases, the exosome is isolated from a stromal stem cell is treated with an IRG1/ACOD1 plasmid. In some cases, the exosome is isolated from a stromal stem cell is treated with a virus expressing IRG1/ACOD1. In some cases, the exosome is isolated from a stromal stem cell is treated with an IRG1/ACOD1 agonist. In some cases, IRG1/ACOD1 expression is increased by 20% in a treated stromal stem cell compared to an untreated stromal stem cell. In some cases, IRG1/ACOD1 expression or activity is increased by 50% in a treated stromal stem cell compared to an untreated stromal stem cell. In some cases, IRG1/ACOD1 expression or activity is increased by 80% in a treated stromal stem cell compared to an untreated stromal stem cell. In some cases, IRG1/ACOD1 expression or activity is increased by 100% in a treated stromal stem cell compared to an untreated stromal stem cell. In some cases, IRG1/ACOD1 expression or activity is increased by 150% in a treated stromal stem cell compared to an untreated stromal stem cell. In some cases, IRG1/ACOD1 expression or activity is increased by 200% in a treated stromal stem cell compared to an untreated stromal stem cell. In some cases, IRG1/ACOD1 expression or activity is increased by 250% in a treated stromal stem cell compared to an untreated stromal stem cell. In some cases, IRG1/ACOD1 expression or activity is increased by 300% in a treated stromal stem cell compared to an untreated stromal stem cell. In some cases, IRG1/ACOD1 expression or activity is increased by 350% in a treated stromal stem cell compared to an untreated stromal stem cell. In some cases, IRG1/ACOD1 expression or activity is increased by 400% in a treated stromal stem cell compared to an untreated stromal stem cell. In some cases, IRG1/ACOD1 expression or activity is increased by 450% in a treated stromal stem cell compared to an untreated stromal stem cell. In some cases, IRG1/ACOD1 expression or activity is increased by 500% in a treated stromal stem cell compared to an untreated stromal stem cell. In some cases, stromal stem cells are isolated from bone marrow or umbilical cord tissue.

In certain aspects, described herein are compositions for increasing monocyte and macrophage polarization to M2 phenotype comprising an exosome treated to decrease expression of or inhibit CD47. In some cases, the exosome is an SDC2+ exosome. In some cases, the exosome is isolated from a stromal stem cell is treated with a CD47 siRNA. In some cases, the exosome is isolated from a stromal stem cell is treated with a virus expressing an shRNA targeting CD47. In some cases, the exosome is isolated from a stromal stem cell is treated with a CD47 inhibitor. In some cases, CD47 expression is reduced by 10% in a treated stromal stem cell compared to an untreated stromal stem cell. In some cases, CD47 expression or activity is reduced by 20% in a treated stromal stem cell compared to an untreated stromal stem cell. In some cases, CD47 expression or activity is reduced by 30% in a treated stromal stem cell compared to an untreated stromal stem cell. In some cases, CD47 expression or activity is reduced by 40% in a treated stromal stem cell compared to an untreated stromal stem cell. In some cases, CD47 expression or activity is reduced by 50% in a treated stromal stem cell compared to an untreated stromal stem cell. In some cases, CD47 expression or activity is reduced by 60% in a treated stromal stem cell compared to an untreated stromal stem cell. In some cases, CD47 expression or activity is reduced by 70% in a treated stromal stem cell compared to an untreated stromal stem cell. In some cases, CD47 expression or activity is reduced by 80% in a treated stromal stem cell compared to an untreated stromal stem cell. In some cases, CD47 expression or activity is reduced by 90% in a treated stromal stem cell compared to an untreated stromal stem cell. In some cases, CD47 expression or activity is reduced by 95% in a treated stromal stem cell compared to an untreated stromal stem cell. In some cases, CD47 expression or activity is reduced by 99% in a treated stromal stem cell compared to an untreated stromal stem cell. In some cases, CD47 expression or activity is reduced by 100% in a treated stromal stem cell compared to an untreated stromal stem cell. In some cases, stromal stem cells are isolated from bone marrow or umbilical cord tissue.

In certain aspects, described herein are compositions for increasing monocyte and macrophage polarization to M2 phenotype comprising an exosome treated to increase expression of or activate PGC1α. In some cases, the exosome is an SDC2+ exosome. In some cases, the exosome is isolated from a stromal stem cell is treated with a PGC1α plasmid. In some cases, the exosome is isolated from a stromal stem cell is treated with a virus expressing PGC1α. In some cases, the exosome is isolated from a stromal stem cell is treated with a PGC1α agonist. In some cases, PGC1α expression is increased by 20% in a treated stromal stem cell compared to an untreated stromal stem cell. In some cases, PGC1α expression or activity is increased by 50% in a treated stromal stem cell compared to an untreated stromal stem cell. In some cases, PGC1α expression or activity is increased by 80% in a treated stromal stem cell compared to an untreated stromal stem cell. In some cases, PGC1α expression or activity is increased by 100% in a treated stromal stem cell compared to an untreated stromal stem cell. In some cases, PGC1α expression or activity is increased by 150% in a treated stromal stem cell compared to an untreated stromal stem cell. In some cases, PGC1α expression or activity is increased by 200% in a treated stromal stem cell compared to an untreated stromal stem cell. In some cases, PGC1α expression or activity is increased by 250% in a treated stromal stem cell compared to an untreated stromal stem cell. In some cases, PGC1α expression or activity is increased by 300% in a treated stromal stem cell compared to an untreated stromal stem cell. In some cases, PGC1α expression or activity is increased by 350% in a treated stromal stem cell compared to an untreated stromal stem cell. In some cases, PGC1α expression or activity is increased by 400% in a treated stromal stem cell compared to an untreated stromal stem cell. In some cases, PGC1α expression or activity is increased by 450% in a treated stromal stem cell compared to an untreated stromal stem cell. In some cases, PGC1α expression or activity is increased by 500% in a treated stromal stem cell compared to an untreated stromal stem cell. In some cases, stromal stem cells are isolated from bone marrow or umbilical cord tissue.

Compositions comprising stromal stem cells for mitochondrial transfer are disclosed herein. Some compositions comprise stromal stem cells modified to have reduced CDC50 and/or CD47 activity or expression and/or increased IRG1/ACOD1 and/or PGC1α activity or expression in a therapeutically effective amount. In some compositions, the amount of modified stromal stem cells ranges from 10^3-10^8 modified stromal stem cells, for example 10^3, 10^4, 10^5, 10^6, 10^7, or 10^8 modified stromal stem cells, or more modified stromal stem cells in the composition. Modified stromal stem cell compositions, in some cases, are concentrated to be diluted by the individual or the health care provider prior to administration. In some cases, modified stromal stem cell compositions are diluted and ready to be administered by the individual or health care provider. In some cases, modified stromal stem cell compositions are contained in single use vials, syringes, or IV bags. In some cases, multiple doses are present in a single container.

Stromal stem cell compositions for increasing monocyte and macrophage polarization to M2 phenotype disclosed herein, in some cases, comprise a cryoprotectant or cryopreservative. Cryoprotectants include DMSO, glycerol, polyethylene glycol, propylene glycol, glycerine, polyvinylpyrolidone, sorbitol, dextran, trehalose, and commercial formulations such as CryoStor from Biolife solutions. Stromal stem cell compositions herein retain potency after being frozen using special freezing protocols. Special freezing protocols include flash freezing, programmable rate freezer, and freezing in an insulated container. The stromal stem cell compositions are in some cases frozen in buffer or culture media having an added cryoprotectant. Buffers include physiologically acceptable buffers such as phosphate buffer, histidine buffer, citrate buffer, acetate buffer, Hypothermasol from Biolife Solutions and other suitable.

Stromal stem cell compositions for increasing monocyte and macrophage polarization to M2 phenotype disclosed herein are formulated in a physiologically acceptable buffer and in some cases supplemented by at least one excipient. Non-limiting examples of excipients include sucrose, trehalose, polyethylene glycol, a polysaccharide, a carrier protein, an inert protein, dextran, hydroxyl ethyl starch (HETA), PEG-4000, gelatin, PLGA, Eudragit RS 100 Nanoparticles, and combinations thereof. Such stromal stem cell compositions are stored at a temperature determined to be most stable (i.e., wherein the stromal stem cell composition retains highest potency, or retains potency for the longest period of time, or otherwise optimizes a desired trait). In some cases, addition of at least one excipient allows the composition to retain potency, such as paracrine signaling potency, when stored at a higher temperature than otherwise would be possible.

While exosome compositions for increasing monocyte and macrophage polarization to M2 phenotype described herein, in some cases, are derived from cells, the exosome compositions do not necessarily comprise living cells. Cell-free exosome compositions, therefore, are non-tumorigenic, that is, they do not increase the susceptibility of a subject to developing a tumor or cancer, because they do not comprise cells capable of differentiating into tumor cells. In alternative compositions, the exosomes are supplemented with cells, such as mesenchymal stromal cells, that contribute to antiinflammatory activity or paracrine signaling activity of the compositions.

Compositions comprising a wide range of exosomes for increasing monocyte and macrophage polarization to M2 phenotype are disclosed herein. Some compositions comprise exosomes in a therapeutically effective amount. In some compositions, the amount of exosomes ranges from 10⁶-10⁸ exosomes, for example 10⁶, 10⁷, 10⁸, or more exosomes in the composition. In some cases, a therapeutically effective amount of exosomes ranges from 1 µg to 700 mg of exosomes, for example 1 µg, 10 µg, 20 µg, 50 µg, 100 µg, 150 µg, 200 µg, 250 µg, 500 µg, 750 µg, 1 mg, 2 mg, 3 mg, 4 mg, 5 mg, 6 mg, 7 mg, 10 mg, 20 mg, 50 mg, 100 mg, 200 mg, 300 mg, 400 mg, 500 mg, 600 mg, 700 mg, or more exosomes in the composition. Exosome compositions, in some cases, are concentrated to be diluted by the individual prior to administration. In some cases, exosome compositions are diluted and ready to be administered by the individual. In some cases, exosome compositions are contained in single use vials or syringes. In some cases, multiple doses are present in a single container.

Exosome compositions for increasing monocyte and macrophage polarization to M2 phenotype disclosed herein retain potency or activity, such as paracrine signaling activity, after being frozen or cryopreserved, In some cases without the use of a cryoprotectant. Cryoprotectants include DMSO, glycerol, polyethylene glycol, propylene glycol, glycerine, polyvinylpyrolidone, sorbitol, dextran, trehalose, and commercial formulations such as CryoStor from Biolife solutions. The exosome compositions also retain potency after being frozen without using special freezing protocols. Special freezing protocols include flash freezing, programmable rate freezer, and freezing in an insulated container. A benefit of the durability of the exosome compositions is that they are more easily frozen and are frozen without cryoprotectants, resulting in compositions that are more durable, more easily and cheaply made, and less likely to suffer from batch variation resulting from loss of activity due to a defect in freezing protocol or composition. The exosome compositions are in some cases frozen in buffer or culture media. Buffers include physiologically acceptable buffers such as phosphate buffer, histidine buffer, citrate buffer, acetate buffer, Hypothermasol from Biolife Solutions and other suitable buffers. In some cases exosome compositions disclosed herein are lyophilized.

Exosome compositions for increasing monocyte and macrophage polarization to M2 phenotype disclosed herein are formulated in a physiologically acceptable buffer and in some cases supplemented by at least one excipient. Non-limiting examples of excipients include sucrose, trehalose, polyethylene glycol, a polysaccharide, a carrier protein, an inert protein, dextran, hydroxyl ethyl starch (HETA), PEG-4000, gelatin, PLGA, Eudragit RS 100 Nanoparticles, and combinations thereof. Such exosome compositions are stored at a temperature determined to be most stable (i.e., wherein the exosome composition retains highest potency, or retains potency for the longest period of time, or otherwise optimizes a desired trait). In some cases, addition of at least one excipient allows the composition to retain potency, such as paracrine signaling potency, when stored at a higher temperature than otherwise would be possible.

### Methods of Preparing Compositions for Monocyte/Macrophage Polarization

Provided herein are methods of preparing compositions comprising stromal stem cells and exosome compositions having increased capabilities of monocyte and macrophage polarization to M2 phenotype, such as SDC2+ stromal stem cells and SDC2+ exosomes treated to have at least one of increased mitochondrial numbers, increased mitochondrial density, increased cell surface phosphatidylserine, and increased itaconate compared to untreated SDC2+ stromal stem cells and SDC2+ exosomes.

Methods herein for preparing compositions for increasing monocyte and macrophage polarization to M2 phenotype comprising stromal stem cells and exosomes, including SDC2+ stromal stem cells and SDC2+ exosomes, comprise treating stromal stem cells to increase their capacity for mitochondrial transfer. Non-limiting examples of methods for preparing compositions for mitochondrial transfer comprising SDC2+ stromal stem cells and SDC2+ exosomes include but are not limited to methods of decreasing expression of CDC50, methods of inhibiting CDC50 activity, methods of increasing expression of IRG1/ACOD1, and methods of increasing IRG1/ACOD1 activity, methods of decreasing expression of CD47, methods of inhibiting CD47, methods of increasing expression of PGC1α, and methods of increasing PGC1α activity, including combinations of the above methods. In some cases, methods for preparing compositions for mitochondrial transfer comprising SDC2+ stromal stem cells and SDC2+ exosomes comprise treating to decrease expression of CDC50 or comprise treating to inhibit CDC50 activity. Alternatively or in combination, methods for preparing compositions for mitochondrial transfer comprising SDC2+ stromal stem cells and SDC2+ exosomes comprise treating to increase expression of IRG1/ACOD1 or comprise treating to increase IRG1/ACOD1 activity. Alternatively or in combination, methods for preparing compositions for mitochondrial transfer comprising SDC2+ stromal stem cells and SDC2+ exosomes comprise treating to decrease expression of CD47 or comprise treating to inhibit CD47 activity. Alternatively or in combination, methods for preparing compositions for mitochondrial transfer comprising SDC2+ stromal stem cells and SDC2+ exosomes comprise treating to increase expression of PGC1α or comprise treating to increase PGC1α activity. Exemplary methods for treating to decrease expression of CDC50 and/or CD47 include but are not limited to gene knockout and RNAi methods, using CRISPR or siRNA techniques suitable for methods herein. Exemplary methods for treating to increase expression of PGC1α include but are not limited to transfecting or transducing the cells with a plasmid or a virus that overexpresses PGC1α.

In certain aspects, provided herein are methods for preparing compositions for increasing monocyte and macrophage polarization to M2 phenotype comprising a stromal stem cell, the method comprising treating a stromal stem cell to decrease expression of or inhibit CDC50. In some cases, the stromal stem cell is an SDC2+ stromal stem cell. In some cases, the method comprises treating the stromal stem cell with a CDC50 siRNA. In some cases, the method comprises treating the stromal stem cell with a virus expressing an shRNA targeting CDC50. In some cases, the method comprises treating the stromal stem cell with a CDC50 inhibitor. In some cases, CDC50 expression is reduced by 10% in a treated stromal stem cell compared to an untreated stromal stem cell. In some cases, CDC50 expression or activity is reduced by 20% in a treated stromal stem cell compared to an untreated stromal stem cell. In some cases, CDC50 expression or activity is reduced by 30% in a treated stromal stem cell compared to an untreated stromal stem cell. In some cases, CDC50 expression or activity is reduced by 40% in a treated stromal stem cell compared to an untreated stromal stem cell. In some cases, CDC50 expression or activity is reduced by 50% in a treated stromal stem cell compared to an untreated stromal stem cell. In some cases, CDC50 expression or activity is reduced by 60% in a treated stromal stem cell compared to an untreated stromal stem cell. In some cases, CDC50 expression or activity is reduced by 70% in a treated stromal stem cell compared to an untreated stromal stem cell. In some cases, CDC50 expression or activity is reduced by 80% in a treated stromal stem cell compared to an untreated stromal stem cell. In some cases, CDC50 expression or activity is reduced by 90% in a treated stromal stem cell compared to an untreated stromal stem cell. In some cases, CDC50 expression or activity is reduced by 95% in a treated stromal stem cell compared to an untreated stromal stem cell. In some cases, CDC50 expression or activity is reduced by 99% in a treated stromal stem cell compared to an untreated stromal stem cell. In some cases, CDC50 expression or activity is reduced by 100% in a treated stromal stem cell compared to an untreated stromal stem cell.

In further aspects, described herein are methods for preparing compositions for increasing monocyte and macrophage polarization to M2 phenotype comprising a stromal stem cell, the method comprising treating the stromal stem cell to increase expression of or activate IRG1/ACOD1. In some cases, the stromal stem cell is an SDC2+ stromal stem cell. In some cases, the method comprises treating the stromal stem cell with an IRG1/ACOD1 plasmid. In some cases, the method comprises treating the stromal stem cell with a virus expressing IRG1/ACOD1. In some cases, the method comprises treating the stromal stem cell with an IRG1/ACOD1 agonist. In some cases, IRG1/ACOD1 expression is increased by 20% in a treated stromal stem cell compared to an untreated stromal stem cell. In some cases, IRG1/ACOD1 expression or activity is increased by 50% in a treated stromal stem cell compared to an untreated stromal stem cell. In some cases, IRG1/ACOD1 expression or activity is increased by 80% in a treated stromal stem cell compared to an untreated stromal stem cell. In some cases, IRG1/ACOD1 expression or activity is increased by 100% in a treated stromal stem cell compared to an untreated stromal stem cell. In some cases, IRG1/ACOD1 expression or activity is increased by 150% in a treated stromal stem cell compared to an untreated stromal stem cell. In some cases, IRG1/ACOD1 expression or activity is increased by 200% in a treated stromal stem cell compared to an untreated stromal stem cell. In some cases, IRG1/ACOD1 expression or activity is increased by 250% in a treated stromal stem cell compared to an untreated stromal stem cell. In some cases, IRG1/ACOD1 expression or activity is increased by 300% in a treated stromal stem cell compared to an untreated stromal stem cell. In some cases, IRG1/ACOD1 expression or activity is increased by 350% in a treated stromal stem cell compared to an untreated stromal stem cell. In some cases, IRG1/ACOD1 expression or activity is increased by 400% in a treated stromal stem cell compared to an untreated stromal stem cell. In some cases, IRG1/ACOD1 expression or activity is increased by 450% in a treated stromal stem cell compared to an untreated stromal stem cell. In some cases, IRG1/ACOD1 expression or activity is increased by 500% in a treated stromal stem cell compared to an untreated stromal stem cell.

In additional aspects, described herein are methods for preparing compositions for increasing monocyte and macrophage polarization to M2 phenotype comprising a stromal stem cell, the method comprising treating a stromal stem cell to decrease expression of or inhibit CD47. In some cases, the stromal stem cell is an SDC2+ stromal stem cell. In some cases, the method comprises treating the stromal stem cell with a CD47 siRNA. In some cases, the method comprises treating the stromal stem cell with a virus expressing an shRNA targeting CD47. In some cases, the method comprises treating the stromal stem cell is treated with a CD47 inhibitor. In some cases, CD47 expression is reduced by 10% in a treated stromal stem cell compared to an untreated stromal stem cell. In some cases, CD47 expression or activity is reduced by 20% in a treated stromal stem cell compared to an untreated stromal stem cell. In some cases, CD47 expression or activity is reduced by 30% in a treated stromal stem cell compared to an untreated stromal stem cell. In some cases, CD47 expression or activity is reduced by 40% in a treated stromal stem cell compared to an untreated stromal stem cell. In some cases, CD47 expression or activity is reduced by 50% in a treated stromal stem cell compared to an untreated stromal stem cell. In some cases, CD47 expression or activity is reduced by 60% in a treated stromal stem cell compared to an untreated stromal stem cell. In some cases, CD47 expression or activity is reduced by 70% in a treated stromal stem cell compared to an untreated stromal stem cell. In some cases, CD47 expression or activity is reduced by 80% in a treated stromal stem cell compared to an untreated stromal stem cell. In some cases, CD47 expression or activity is reduced by 90% in a treated stromal stem cell compared to an untreated stromal stem cell. In some cases, CD47 expression or activity is reduced by 95% in a treated stromal stem cell compared to an untreated stromal stem cell. In some cases, CD47 expression or activity is reduced by 99% in a treated stromal stem cell compared to an untreated stromal stem cell. In some cases, CD47 expression or activity is reduced by 100% in a treated stromal stem cell compared to an untreated stromal stem cell.

In further aspects, described herein are methods for preparing compositions for increasing monocyte and macrophage polarization to M2 phenotype comprising a stromal stem cell, the method comprising treating the stromal stem cell to increase expression of or activate PGC1α. In some cases, the stromal stem cell is an SDC2+ stromal stem cell. In some cases, the method comprises treating the stromal stem cell with a PGC1α plasmid. In some cases, the method comprises treating the stromal stem cell with a virus expressing PGC1α. In some cases, the method comprises treating the stromal stem cell with a PGC1α agonist. In some cases, PGC1α expression is increased by 20% in a treated stromal stem cell compared to an untreated stromal stem cell. In some cases, PGC1α expression or activity is increased by 50% in a treated stromal stem cell compared to an untreated stromal stem cell. In some cases, PGC1α expression or activity is increased by 80% in a treated stromal stem cell compared to an untreated stromal stem cell. In some cases, PGC1α expression or activity is increased by 100% in a treated stromal stem cell compared to an untreated stromal stem cell. In some cases, PGC1α expression or activity is increased by 150% in a treated stromal stem cell compared to an untreated stromal stem cell. In some cases, PGC1α expression or activity is increased by 200% in a treated stromal stem cell compared to an untreated stromal stem cell. In some cases, PGC1α expression or activity is increased by 250% in a treated stromal stem cell compared to an untreated stromal stem cell. In some cases, PGC1α expression or activity is increased by 300% in a treated stromal stem cell compared to an untreated stromal stem cell. In some cases, PGC1α expression or activity is increased by 350% in a treated stromal stem cell compared to an untreated stromal stem cell. In some cases, PGC1α expression or activity is increased by 400% in a treated stromal stem cell compared to an untreated stromal stem cell. In some cases, PGC1α expression or activity is increased by 450% in a treated stromal stem cell compared to an untreated stromal stem cell. In some cases, PGC1α expression or activity is increased by 500% in a treated stromal stem cell compared to an untreated stromal stem cell.

In certain aspects, provided herein are methods of preparing compositions for increasing monocyte and macrophage polarization to M2 phenotype comprising an exosome the method comprising treating a stromal stem cell to decrease expression of or inhibit CDC50. In some cases, the exosome is an SDC2+ exosome. In some cases, the method comprises isolating the exosome from a stromal stem cell treated with a CDC50 siRNA. In some cases, the method comprises isolating the exosome from a stromal stem cell treated with a virus expressing an shRNA targeting CDC50. In some cases, the method comprises isolating the exosome from a stromal stem cell treated with a CDC50 inhibitor.

In certain aspects, described herein are methods for preparing compositions for increasing monocyte and macrophage polarization to M2 phenotype comprising an exosome treated to increase expression of or activate IRG1/ACOD1. In some cases, the exosome is an SDC2+ exosome. In some cases, the exosome is isolated from a stromal stem cell is treated with a IRG1/ACOD1 plasmid. In some cases, the exosome is isolated from a stromal stem cell is treated with a virus IRG1/ACOD1 IRG1/ACOD1. In some cases, the exosome is isolated from a stromal stem cell is treated with a PGC1α agonist.

In certain aspects, described herein are methods for preparing compositions for increasing monocyte and macrophage polarization to M2 phenotype comprising an exosome treated to decrease expression of or inhibit CD47. In some cases, the exosome is an SDC2+ exosome. In some cases, the exosome is isolated from a stromal stem cell is treated with a CD47 siRNA. In some cases, the exosome is isolated from a stromal stem cell is treated with a virus expressing an shRNA targeting CD47. In some cases, the exosome is isolated from a stromal stem cell is treated with a CD47 inhibitor.

In certain aspects, described herein are methods for preparing compositions for increasing monocyte and macrophage polarization to M2 phenotype comprising an exosome treated to increase expression of or activate PGC1α. In some cases, the exosome is an SDC2+ exosome. In some cases, the exosome is isolated from a stromal stem cell is treated with a PGC1α plasmid. In some cases, the exosome is isolated from a stromal stem cell is treated with a virus expressing PGC1α. In some cases, the exosome is isolated from a stromal stem cell is treated with a PGC1α agonist.

### Methods of Monocyte/Macrophage Polarization

Provided herein are methods of increasing monocyte and macrophage polarization to M2 phenotype comprising administering compositions comprising stromal stem cells and exosome compositions having increased capabilities of monocyte and macrophage polarization to M2 phenotype, such as SDC2+ stromal stem cells and SDC2+ exosomes treated to have at least one of increased mitochondrial numbers, increased mitochondrial density, increased cell surface phosphatidylserine, and increased itaconate compared to untreated SDC2+ stromal stem cells and SDC2+ exosomes. In some cases, stromal stem cells and exosomes for methods herein have increased capacity of transferring mitochondria to a target cell.

Methods of increasing monocyte and macrophage polarization to M2 phenotype in an individual described herein, in certain aspects, comprise intracellularly administering mitochondria to cells of the individual. In some cases, methods of mitochondrial transfer to an individual comprise contacting cells of the individual to a stromal stem cell, such as an SDC2+ stromal stem cell. In some embodiments of methods of mitochondrial transfer herein, stromal stem cells have reduced CD50 activity. In some embodiments, stromal stem cells have increased IRG1/ACOD1 activity. Alternatively or in combination, stromal stem cells have reduced CD47 activity. In some embodiments, stromal stem cells have increased PGC1α activity. In some embodiments, stromal stem cells for methods of mitochondrial transfer herein have one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

Methods of increasing monocyte and macrophage polarization to M2 phenotype in an individual described herein, in certain aspects, comprise intracellularly administering mitochondria to cells of the individual. In some cases, methods of mitochondrial transfer to an individual comprise contacting cells of the individual to an exosome isolated from a stromal stem cell, such as an SDC2+ stromal stem cell. In some embodiments of methods of mitochondrial transfer herein, stromal stem cells have reduced CD50 activity. In some embodiments, stromal stem cells have increased IRG1/ACOD1 activity. Alternatively or in combination, stromal stem cells have reduced CD47 activity. In some embodiments, stromal stem cells have increased PGC1α activity. In some embodiments, exosomes for methods of mitochondrial transfer herein are isolated from stromal stem cells having one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

In methods of increasing monocyte and macrophage polarization to M2 phenotype in an individual and methods of treatment described herein, increasing monocyte and macrophage polarization to M2 phenotype in cells from the individual improves a symptom of a disease. In some cases, the individual has at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

### Methods of Mitochondrial Transfer

Described herein are methods of mitochondrial transfer comprising administering compositions comprising stromal stem cells and exosome compositions having increased capabilities of mitochondrial transfer, such as SDC2+ stromal stem cells and SDC2+ exosomes treated to have at least one of increased mitochondrial numbers, increased mitochondrial density, increased cell surface phosphatidylserine, and increased itaconate compared to untreated SDC2+ stromal stem cells and SDC2+ exosomes. In some cases, stromal stem cells and exosomes for methods herein have increased capacity of transferring mitochondria to a target cell.

Methods of mitochondrial transfer to an individual described herein, in certain aspects, comprise intracellularly administering mitochondria to cells of the individual. In some cases, methods of mitochondrial transfer to an individual comprise contacting cells of the individual to a stromal stem cell, such as an SDC2+ stromal stem cell. In some embodiments of methods of mitochondrial transfer herein, stromal stem cells have reduced CD50 activity. In some embodiments, stromal stem cells have increased IRG1/ACOD1 activity. Alternatively or in combination, stromal stem cells have reduced CD47 activity. In some embodiments, stromal stem cells have increased PGC1α activity. In some embodiments, stromal stem cells for methods of mitochondrial transfer herein have one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

Methods of mitochondrial transfer to an individual described herein, in certain aspects, comprise intracellularly administering mitochondria to cells of the individual. In some cases, methods of mitochondrial transfer to an individual comprise contacting cells of the individual to an exosome isolated from a stromal stem cell, such as an SDC2+ stromal stem cell. In some embodiments of methods of mitochondrial transfer herein, stromal stem cells have reduced CD50 activity. In some embodiments, stromal stem cells have increased IRG1/ACOD1 activity. Alternatively or in combination, stromal stem cells have reduced CD47 activity. In some embodiments, stromal stem cells have increased PGC1α activity. In some embodiments, exosomes for methods of mitochondrial transfer herein are isolated from stromal stem cells having one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

Further described herein are methods of treating a disease in an individual comprising administering to the individual a population of mammalian stromal stem cells, wherein the population of stromal stem cells has reduced expression of CDC50. Alternatively or in combination, the population of mammalian stromal stem cells has increased IRG1/ACOD1 expression or activity. In some cases, the population of mammalian stromal stem cells has reduced CD47 expression or activity. Alternatively or in combination, the population of mammalian stromal stem cells has increased PGC1α expression or activity. In some embodiments, stromal stem cells for methods of treatment herein have one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

Additionally described herein are methods of treating a disease in an individual comprising administering to the individual a population of mammalian stromal stem cells, wherein the population of stromal stem cells has reduced expression of CD47. In some cases, the population of mammalian stromal stem cells has reduced CDC50 expression or activity. Alternatively or in combination, the population of mammalian stromal stem cells has increased IRG1/ACOD1 expression or activity. Alternatively or in combination, the population of mammalian stromal stem cells has increased PGC1α expression or activity. In some embodiments, stromal stem cells for methods of treatment herein have one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

Further described herein are methods of treating a disease in an individual comprising administering to the individual a population of mammalian stromal stem cells, wherein the population of stromal stem cells has increased expression of PGC1α. In some cases, the population of mammalian stromal stem cells has reduced CD47 expression or activity. Alternatively or in combination, the population of mammalian stromal stem cells has reduced CDC50 expression or activity. Alternatively or in combination, the population of mammalian stromal stem cells has increased IRG1/ACOD1 expression or activity. In some embodiments, stromal stem cells for methods of treatment herein have one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

Further provided herein are methods of treating a disease in an individual comprising administering to the individual exosomes isolated from a population of mammalian stromal stem cells, wherein the population of stromal stem cells has reduced expression of CDC50. Alternatively or in combination, the population of mammalian stromal stem cells has increased IRG1/ACOD1 expression or activity. In some cases, the population of mammalian stromal stem cells has reduced CD47 expression or activity. Alternatively or in combination, the population of mammalian stromal stem cells has increased PGC1α expression or activity. In some embodiments, stromal stem cells for methods of treatment herein have one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

Additionally described herein are methods of treating a disease in an individual comprising administering to the individual exosomes isolated from a population of mammalian stromal stem cells, wherein the population of stromal stem cells has reduced expression of CD47. In some cases, the population of mammalian stromal stem cells has reduced CDC50 expression or activity. Alternatively or in combination, the population of mammalian stromal stem cells has increased IRG1/ACOD1 expression or activity. Alternatively or in combination, the population of mammalian stromal stem cells has increased PGC1α expression or activity. In some embodiments, stromal stem cells for methods of treatment herein have one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

Further described herein are methods of treating a disease in an individual comprising administering to the individual exosomes isolated from a population of mammalian stromal stem cells, wherein the population of stromal stem cells has increased expression of PGC1α. Alternatively or in combination, the population of mammalian stromal stem cells has reduced CDC50 expression or activity. Alternatively or in combination, the population of mammalian stromal stem cells has reduced IRG1/ACOD1 expression or activity. In some cases, the population of mammalian stromal stem cells has reduced CD47 expression or activity. In some embodiments, stromal stem cells for methods of treatment herein have one or more of reduced CD50 activity, increased IRG1/ACOD1 activity, reduced CD47 activity, and increased PGC1α activity. In some cases, stromal stem cells are at least 30% positive for SDC2. In some cases, CDC50 expression or activity is reduced by treating the stromal stem cell with at least one of a CDC50 antagonist, a CDC50 inhibitory nucleic acid, and a CDC50 inhibitory peptide. Alternatively or in combination, IRG1/ACOD1 expression or activity is increased by treating the stromal stem cell with at least one of a IRG1/ACOD1 agonist and a nucleic acid encoding IRG1/ACOD1. Alternatively or in combination, CD47 expression or activity is reduced by treating the stromal stem cell with at least one of a CD47 antagonist, a CD47 inhibitory nucleic acid, and a CD47 inhibitory peptide. Alternatively or in combination, PGC1α expression or activity is increased by treating the stromal stem cell with at least one of a PGC1α agonist and a nucleic acid encoding PGC1α. In some embodiments, stromal stem cells have an increased total number of mitochondria. In some embodiments, stromal stem cells have an increased total volume of mitochondria. In some embodiments, stromal stem cells have increased cell surface phosphatidylserine. In some embodiments, stromal stem cells have increased itaconate.

In methods of mitochondrial transfer to an individual and methods of treatment described herein, transfer of mitochondria to cells from the individual improves a symptom of a disease. In some cases, the individual has at least one symptom of a disease selected from the group consisting of a liver disease, a diabetes, a diabetes related disease, a cancer, an acute lung injury, an acute respiratory distress syndrome, a chronic obstructive pulmonary disorder, an idiopathic pulmonary fibrosis, a sepsis, a bone marrow transplant, a hematopoietic stem cell rejection, a solid organ transplant rejection, an acute toxin induced liver failure, an autoimmune hepatitis, a primary biliary cirrhosis, a primary sclerosing cholangitis, an osteonecrosis, a degenerative disc disease, a rheumatoid arthritis, an osteoarthritis, an autoimmune nephritis, a Wegener's granulomatosis, a burn, a muscle wasting condition, an atrophic syndrome, a sarcopenia, a cachexia, a muscular dystrophy, a congestive heart failure, an acute myocardial infarction, a stroke, a retinopathy, a nephropathy, a myopathy, an atherosclerosis, a peripheral artery disease, a critical limb ischemia, a uveitis, a macular degeneration, an autoimmune condition, an autoimmune gastritis, a graft-versus-host disease, a multiple sclerosis, a demyelinating disease, a thyroid disease, an inflammatory bowel disease, a Crohn's disease, an ulcerative colitis, a scleroderma, a lupus, a Graves' disease, an autoimmune lyphoproliferative disease, a laminitis, a tendon injury, and an exercise induced pulmonary haemorrhage.

### EXAMPLES

The following examples are given for the purpose of illustrating various embodiments of the invention and are not meant to limit the present invention in any fashion. The present examples, along with the methods described herein are presently representative of preferred embodiments, are exemplary, and are not intended as limitations on the scope of the invention.

### Example 1: Preparation of Improved SDC2+ Stromal Stem Cells

Stromal stem cells are isolated from human umbilical cord tissue. The cells are treated with a transgene expressing a shRNA for downregulating CDC50 and CD47 and a second transgene expressing PGC1α. The treated cells are then sorted for cells expressing SDC2. A second population of stromal stem cells was treated with empty vector and sorted for cells expressing SDC2. The treated stromal stem cells are observed to have increased mitochondrial numbers as well as increased phosphatidylserine (PS) on the surface of the cell compared to the stromal stem cells treated with empty vector. Both populations of stromal stem cells are also observed to have standardized proliferative capacity.

This example shows that SDC2+ stromal stem cells having decreased CDC50 and CD47 and increased PGC1α have increased mitochondrial numbers and increased cell surface PS than control untreated SDC2+ stromal stem cells.

### Example 2: Preparation of Improved SDC2+ Exosomes

Stromal stem cells are isolated from human umbilical cord tissue. The cells are treated with a transgene expressing a shRNA for downregulating CDC50 and CD47 and a second transgene expressing PGC1α. Exosomes from the treated cells are then sorted for exosomes having SDC2. A second population of stromal stem cells was treated with empty vector and exosomes expressing SDC2 are isolated from the stromal stem cells. Exosomes from the treated stromal stem cells are observed to have increased mitochondrial numbers as well as increased phosphatidylserine (PS) on the surface of the exosome compared to exosomes from the stromal stem cells treated with empty vector.

This example shows that exosomes from SDC2+ stromal stem cells having decreased CDC50 and CD47 and increased PGC1α have increased mitochondrial numbers and increased cell surface PS than control exosomes from control untreated SDC2+ stromal stem cells.

### Example 3: In Vitro Mitochondrial Transfer with SDC2+ Stromal Stem Cells

Stromal stem cells are isolated from human umbilical cord tissue. The cells are treated with a transgene expressing a shRNA for downregulating CDC50 and CD47 and a second transgene expressing PGC1α. The treated cells are then sorted for cells expressing SDC2. A second population of stromal stem cells was treated with empty vector and sorted for cells expressing SDC2. The treated stromal stem cells are observed to have increased mitochondrial numbers as well as increased phosphatidylserine (PS) on the surface of the cell compared to the stromal stem cells treated with empty vector. Both populations of stromal stem cells are also observed to have standardized proliferative capacity.

The treated and sorted stromal stem cells are cultured with macrophages. The second population of empty vector treated, sorted stromal stem cells are cultured with a second population of macrophages. Both populations of macrophages are then stained for mitochondrial markers at various time points. The numbers of mitochondria in the macrophages cultured with treated stromal stem cells are observed to increase over time compared with control macrophages cultured in medium only. The numbers of mitochondria in the macrophages cultured with stromal stem cells that are treated with empty vector have an increase in mitochondria over time compared with control macrophages but the effect is reduced compared to macrophages cultured with treated stromal stem cells.

This example shows that SDC2+ stromal stem cells having decreased CDC50 and CD47 and increased PGC1α have increased efficacy in transferring mitochondria to treated cells than control SDC2+ stromal stem cells.

### Example 4: In Vitro Mitochondrial Transfer with SDC2+ Exosomes

Stromal stem cells are isolated from human umbilical cord tissue. The cells are treated with a transgene expressing a shRNA for downregulating CDC50 and CD47 and a second transgene expressing PGC1α. Exosomes from the treated cells are then sorted for exosomes having SDC2. A second population of stromal stem cells was treated with empty vector and exosomes expressing SDC2 are isolated from the stromal stem cells. Exosomes from the treated stromal stem cells are observed to have increased mitochondrial numbers as well as increased phosphatidylserine (PS) on the surface of the exosome compared to exosomes from the stromal stem cells treated with empty vector.

Exosomes from the treated and sorted stromal stem cells are cultured with macrophages. The second population of exosomes from empty vector treated, sorted stromal stem cells are cultured with a second population of macrophages. Both populations of macrophages are then stained for mitochondrial markers at various time points. The numbers of mitochondria in the macrophages cultured with exosomes from treated stromal stem cells are observed to increase over time compared with control macrophages cultured in medium only. The numbers of mitochondria in the macrophages cultured with exosomes from stromal stem cells that are treated with empty vector have an increase in mitochondria over time compared with control macrophages but the effect is reduced compared to macrophages cultured with exosomes from treated stromal stem cells.

This example shows that exosomes from SDC2+ stromal stem cells having decreased CDC50 and CD47 and increased PGC1α have increased efficacy in transferring mitochondria to treated cells than control exosomes from SDC2+ stromal stem cells.

### Example 5: Treatment of Diabetic Ulcers

A composition comprising SDC2+ stromal stem cells that have been treated with siRNA to reduce CDC50 and CD47 and a PGC1α transgene formulated in a collagen matrix is used to treat a group of individuals in need of treatment of diabetic ulcers. After administration of the treated SDC2+ stromal stem cell composition and covering the ulcers with a bandage, the diabetic ulcers are shown to fully heal in 10/10 treated individuals.

A second population of individuals needing treatment for diabetic ulcers is treated with a composition comprising SDC2+ stromal stem cells treated with an empty vector. After administration of the empty-vector treated SDC2+ stromal stem cells and covering the ulcers with a bandage, the diabetic ulcers are shown to fully heal in 7/10 of the treated individuals and decrease in severity in 9/10 of the treated individuals.

This example shows that SDC2+ stromal stem cells having decreased CDC50 and CD47 and increased PGC1α have increased efficacy in treating diabetic ulcers than control SDC2+ stromal stem cells.

### Example 6: Treatment of Diabetic Ulcers

A composition comprising exosomes from SDC2+ stromal stem cells that have been treated with siRNA to reduce CDC50 and CD47 and a PGC1α transgene formulated in a collagen matrix is used to treat a group of individuals in need of treatment of diabetic ulcers. After administration of exosomes from the treated SDC2+ stromal stem cell composition and covering the ulcers with a bandage, the diabetic ulcers are shown to fully heal in 10/10 treated individuals.

A second population of individuals needing treatment for diabetic ulcers is treated with a composition comprising exosomes from SDC2+ stromal stem cells treated with an empty vector. After administration of exosomes from the empty-vector treated SDC2+ stromal stem cells and covering the ulcers with a bandage, the diabetic ulcers are shown to fully heal in 7/10 of the treated individuals and decrease in severity in 9/10 of the treated individuals.

This example shows that exosomes from SDC2+ stromal stem cells having decreased CDC50 and CD47 and increased PGC1α have increased efficacy in treating diabetic ulcers than exosomes from control SDC2+ stromal stem cells.

### Example 7: Treatment of Inflammatory Liver Disease

A composition comprising SDC2+ stromal stem cells that have been treated with siRNA to reduce CDC50 and CD47 and a PGC1α transgene formulated for intravenous administration is used to treat a group of individuals in need of treatment of primary sclerosing cholangitis or autoimmune hepatitis. After administration of the treated SDC2+ stromal stem cell composition, serum inflammatory markers are reduced and liver function markers are increased 10/10 treated individuals.

A second population of individuals needing treatment for primary sclerosing cholangitis or autoimmune hepatitis is treated with a composition comprising SDC2+ stromal stem cells treated with an empty vector. After administration of the empty-vector treated SDC2+ stromal stem cells, serum inflammatory markers are reduced and liver function markers are increased in 7/10 of the treated individuals.

This example shows that SDC2+ stromal stem cells having decreased CDC50 and CD47 and increased PGC1α have increased efficacy in treating primary sclerosing cholangitis or autoimmune hepatitis than control SDC2+ stromal stem cells.

### Example 8: Treatment of Inflammatory Liver Disease

A composition comprising exosomes from SDC2+ stromal stem cells that have been treated with siRNA to reduce CDC50 and CD47 and a PGC1α transgene formulated for intravenous administration is used to treat a group of individuals in need of treatment of primary sclerosing cholangitis or autoimmune hepatitis. After administration of exosomes from the treated SDC2+ stromal stem cell composition, serum inflammatory markers are reduced and liver function markers are increased in 10/10 treated individuals.

A second population of individuals needing treatment for primary sclerosing cholangitis or autoimmune hepatitis is treated with a composition comprising exosomes from SDC2+ stromal stem cells treated with an empty vector. After administration of exosomes from the empty-vector treated SDC2+ stromal stem cells, inflammatory markers are reduced and liver function markers are increased in 7/10 of the treated individuals.

This example shows that exosomes from SDC2+ stromal stem cells having decreased CDC50 and CD47 and increased PGC1α have increased efficacy in treating primary sclerosing cholangitis or autoimmune hepatitis than exosomes from control SDC2+ stromal stem cells.

### Example 9: Treatment of Diabetic Kidney Disease

A composition comprising SDC2+ stromal stem cells that have been treated with siRNA to reduce CDC50 and CD47 and a PGC1α transgene formulated for intravenous administration is used to treat a group of individuals in need of treatment of diabetic kidney disease. After administration of the treated SDC2+ stromal stem cell composition, serum inflammatory markers are reduced and kidney function markers are increased 10/10 treated individuals.

A second population of individuals needing treatment for diabetic kidney disease is treated with a composition comprising SDC2+ stromal stem cells treated with an empty vector. After administration of the empty-vector treated SDC2+ stromal stem cells, serum inflammatory markers are reduced and kidney function markers are increased in 7/10 of the treated individuals.

This example shows that SDC2+ stromal stem cells having decreased CDC50 and CD47 and increased PGC1α have increased efficacy in treating diabetic kidney disease than control SDC2+ stromal stem cells.

### Example 10: Treatment of Diabetic Kidney Disease

A composition comprising exosomes from SDC2+ stromal stem cells that have been treated with siRNA to reduce CDC50 and CD47 and a PGC1α transgene formulated for intravenous administration is used to treat a group of individuals in need of treatment of diabetic kidney disease. After administration of exosomes from the treated SDC2+ stromal stem cell composition, serum inflammatory markers are reduced and kidney function markers are increased in 10/10 treated individuals.

A second population of individuals needing treatment for diabetic kidney disease is treated with a composition comprising exosomes from SDC2+ stromal stem cells treated with an empty vector. After administration of exosomes from the empty-vector treated SDC2+ stromal stem cells, inflammatory markers are reduced and kidney function markers are increased in 7/10 of the treated individuals.

This example shows that exosomes from SDC2+ stromal stem cells having decreased CDC50 and CD47 and increased PGC1α have increased efficacy in treating diabetic kidney disease than exosomes from control SDC2+ stromal stem cells.

### Example 11: Treatment of Chronic Obstructive Pulmonary Disease

A composition comprising SDC2+ stromal stem cells that have been treated with siRNA to reduce CDC50 and CD47 and a PGC1α transgene formulated for pulmonary administration is used to treat a group of individuals in need of treatment of chronic obstructive pulmonary disease. After administration of the treated SDC2+ stromal stem cell composition, serum inflammatory markers are reduced and lung function is increased 10/10 treated individuals.

A second population of individuals needing treatment for chronic obstructive pulmonary disease is treated with a composition comprising SDC2+ stromal stem cells treated with an empty vector. After administration of the empty-vector treated SDC2+ stromal stem cells, serum inflammatory markers are reduced and lung function is increased in 7/10 of the treated individuals.

This example shows that SDC2+ stromal stem cells having decreased CDC50 and CD47 and increased PGC1α have increased efficacy in treating chronic obstructive pulmonary disease than control SDC2+ stromal stem cells.

### Example 12: Treatment of Chronic Obstructive Pulmonary Disease

A composition comprising exosomes from SDC2+ stromal stem cells that have been treated with siRNA to reduce CDC50 and CD47 and a PGC1α transgene formulated for pulmonary administration is used to treat a group of individuals in need of treatment of chronic obstructive pulmonary disease. After administration of exosomes from the treated SDC2+ stromal stem cell composition, serum inflammatory markers are reduced and lung function is increased in 10/10 treated individuals.

A second population of individuals needing treatment for diabetic kidney disease is treated with a composition comprising exosomes from SDC2+ stromal stem cells treated with an empty vector. After administration of exosomes from the empty-vector treated SDC2+ stromal stem cells, inflammatory markers are reduced and lung function is increased in 7/10 of the treated individuals.

This example shows that exosomes from SDC2+ stromal stem cells having decreased CDC50 and CD47 and increased PGC1α have increased efficacy in treating chronic obstructive pulmonary disease than exosomes from control SDC2+ stromal stem cells.

### Example 13: Correlation between the amount of indoleamine 2,3-dioxygenase (IDO) produced and the T cell inhibitory potential of MSC donors

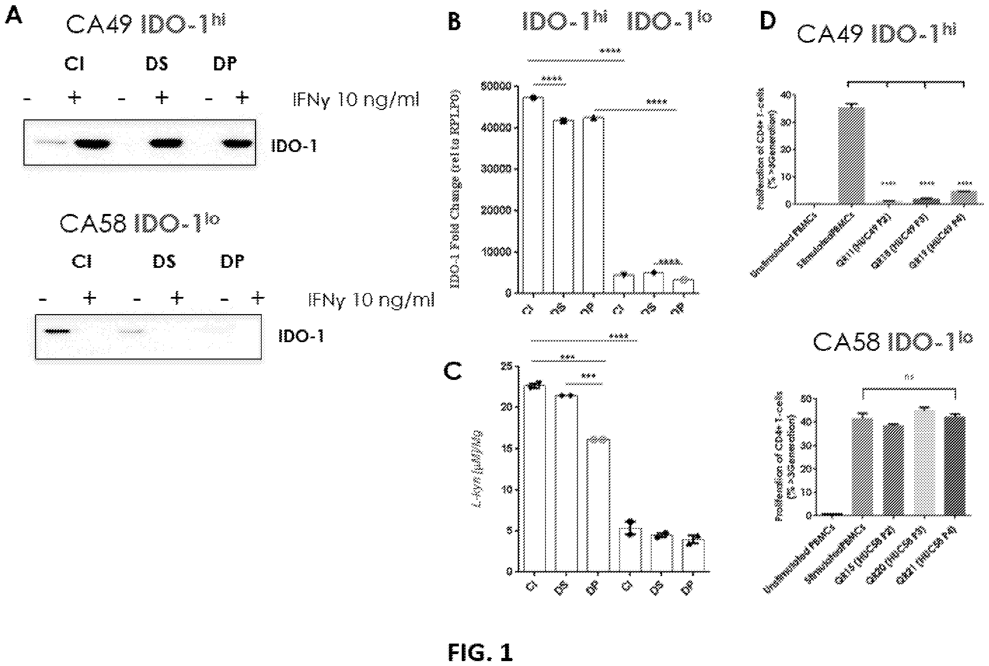

MSCs (CA49 and CA58) were cultured for 24 h, stimulated for 24 h and harvested before total cell lysates were assayed for human IDO. The expression levels of human IDO protein were determined by western blotting analysis as shown in **Fig. 1A****.** Unstimulated MSCs served as a negative control, and human MSCs stimulated with the inflammatory cytokine IFNγ (10 ng/mL) served as positive control. As shown, IDO-1^{hi} DP retains IDO-1 expression.

IDO-1^{hi} (CA49DP) and IDO-1^{lo} (CA58DP) quantum-expanded donors were stimulated with 10 ng/mL for 24 h. The expression level of IDO mRNA relative to RPLP0 was evaluated by Taqman real-time QPCR as shown in **Fig. 1B****.** The Delta-delta CT method was applied to calculate the fold induction and error bars indicate upper and lower limit. Control IDO-1 gene expression following IFNγ (10 ng/ml, 24 h) in an IDO-1hi and IDO-1lo donor.

**Fig. 1C** shows an impact of IFNY on the enzymatic activity of IDO harvested from conditioned media of IDO-1hi and IDO-1lo donors measured by production of kynurenine. As shown, IDO-1^{hi} DP retains the enzymatic activity of IDO compared to IDO-1^{lo}

**Fig. 1D** shows bar graphs of T cell proliferation assay. T cell proliferation was measured in a proliferation assay performed with carboxyfluorescein diacetate succinimidyl ester (CFSE)-labeled PBMC activated with anti-CD3/CD28 antibodies in the presence of either IDO-1^{hi} or IDO-1^{lo} MSC donors. As shown, IDO-1^{hi} DP suppresses PBMC proliferation.

### Example 14: IDO-1^{hi} DP donor is capable of inducing immune regulatory monocytes in comparison to IDO-1^{lo} DP donor in a 24hr monocyte assay

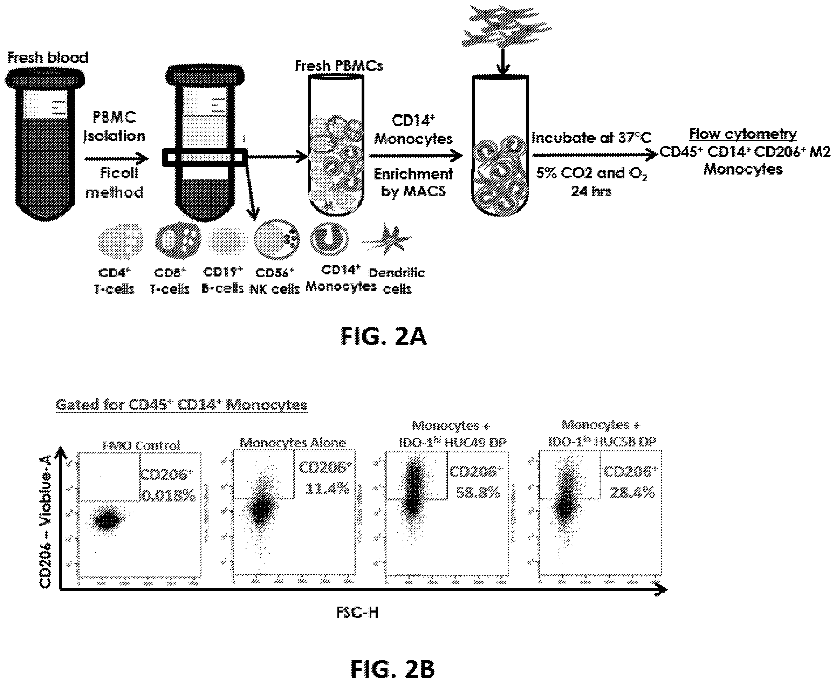

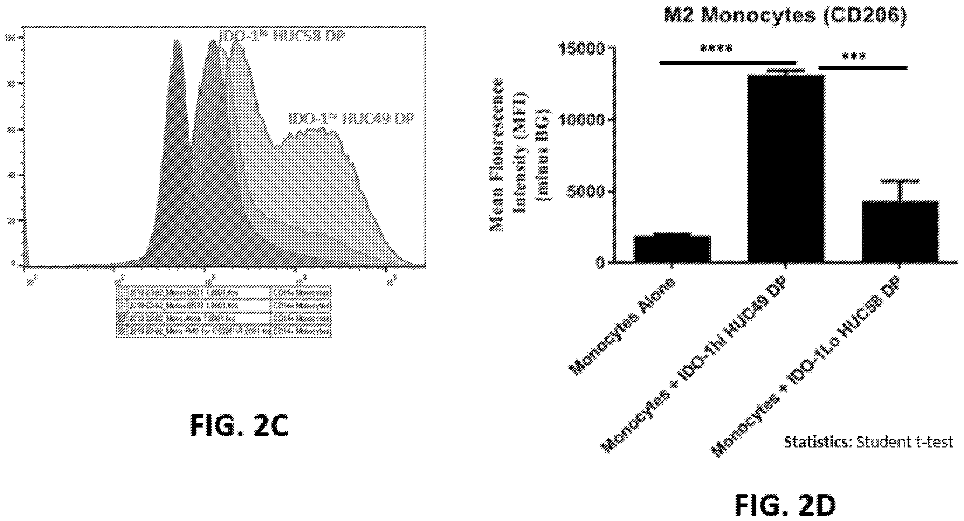

MSCs (ORBCEL-C) were cultured with freshly MACS isolated CD14⁺ monocytes for 24hrs as shown in the schematic diagram of **Fig. 2A****.** In 24 hrs post co-culture of MSC and monocytes, cells were labelled & gated for CD45⁺ CD14⁺ CD206⁺ M2 monocytes. **Fig. 2B** shows FACS assay results of CD206+ populations (control, Monocytes alone, Monocytes + IDO-1^{hi} HUC49 DP (ORBCEL-C), and Monocytes + IDO-1^{lo} HUC58 DP (ORBCEL-C) from left to right). As shown, in Fig. 2C in the histogram depicting the expression of CD206 on cells gated for monocyte population (CD45⁺CD14⁺), CD206+ cell population was increased with co-culture of Monocytes + IDO-1^{hi} HUC49 DP (ORBCEL-C) compared to other groups. Fig. 2D shows representative bar graph showing Mean Fluorescence intensity (MFI) of CD206 expression on monocytes between different conditions (n=4). Statistics: Students T-test.

### Example 15: Reduction in TMEM30A expression results in significantly increased PtS on the cell surface of ORBCEL-C

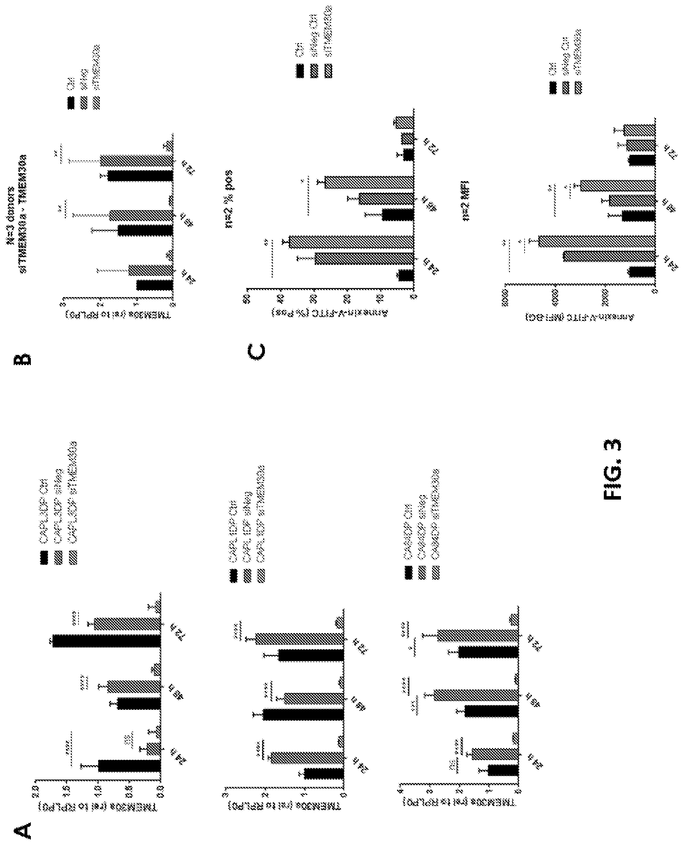

Three independent donor pools of ORBCEL-C were transfected with siRNA directed at TMEM30A or negative siRNA. The expression level of TMEM30A relative to RPLP0 was evaluated by Taqman real-time qPCR. As shown in **Fig. 3A****,** introduction of siRNA directed at TMEM30A significantly reduced the expression of TMEM30A in all three independent donor pools of ORBCEL-C. The Delta-delta CT method was applied to calculate the fold induction and error bars indicates upper and lower limit. **Fig. 3B** shows a combined graph of TMEM30A gene expression of the three pooled ORBCEL-C. **Fig. 3C** shows a graph of flow cytometry (% Pos and MFI) of WT, siTMEM30A and siNegative control cells labelled with annexin V-FITC. As shown, expression of surface phosphatidylserine (PtS) in ORBCEL-C was increased by 48 hours with reduction of TMEM30A expression by siRNA directed at TMEM30A.

### Example 16: Impact of siTMEM30A on ORBCEL-C phenotype

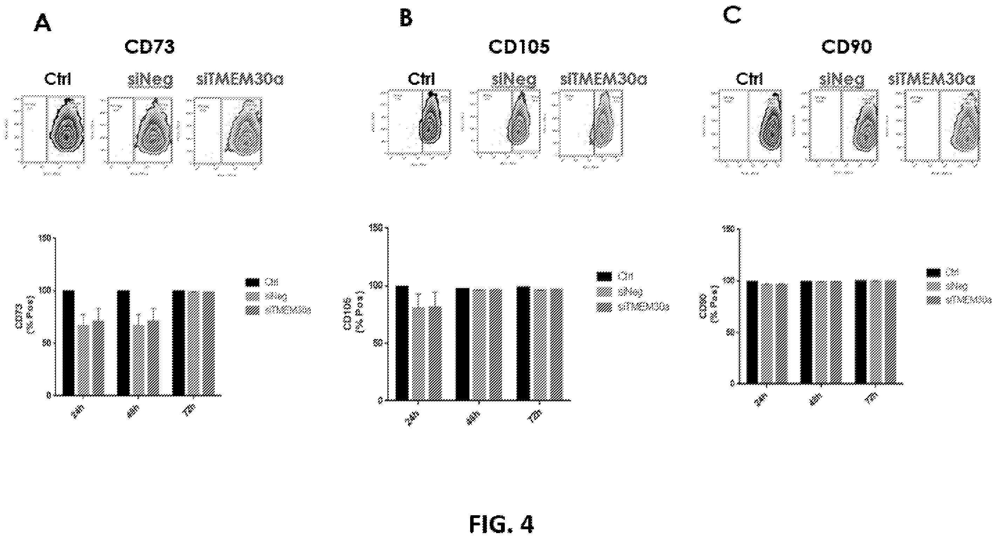

MSCs were plated at 2x10⁵ cells per well in a 6-well plate. Following 24 h cells were transfected with siRNA directed at TMEM30A or negative siRNA and untransfected as controls. Cells were harvested and analysed at 24, 48 and 72 h. A, B and C show % positive for each condition. (Bars are mean ±SEM, One-way ANOVA, n=3. The phenotype of ORBCEL-C^{™} cells were determined by flow cytometric analysis of expression levels of CD73, CD105, and CD90 in the MSCs. As shown in **Figs. 4A-****C**, expression levels of CD73, CD105, and CD90 in the MSCs did not change substantially by inhibiting TMEM30A expression.

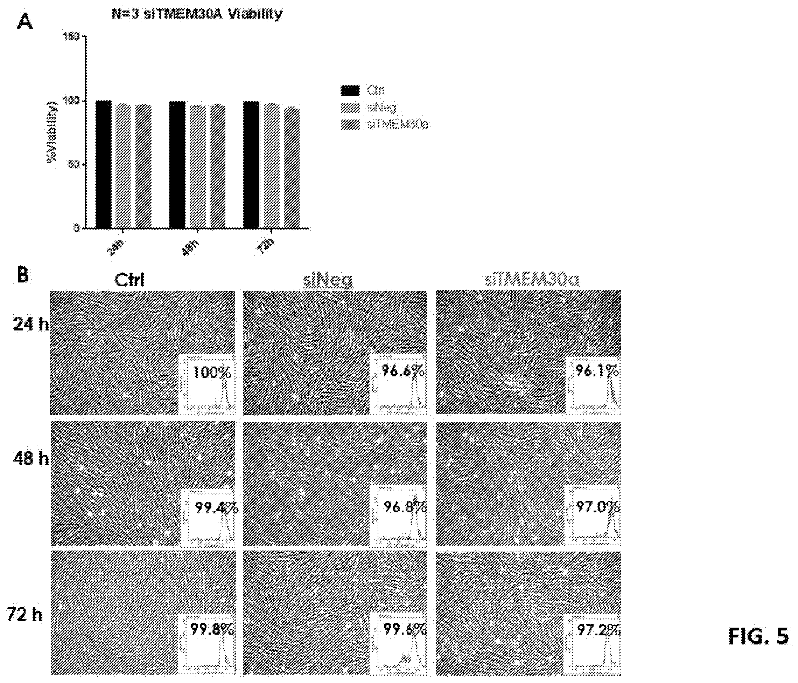

Also, at 24, 48 and 72 hours after transfection, cells were harvested and viability was measured by both NC200 (acridine orange and DAPI stain) and staining with Sytox blue and analyzing by flow cytometry. As shown in **Fig. 5A****,** cell viability of ORBCEL-C was not affected in 24 h, 48h, or 72h after the treatment with siRNA directed at TMEM30A. **Fig. 5B** shows exemplary and representative phase contrast images of MSCs transfected with siTMEM30A that show no change in morphology, density, or adherence.

### Example 17: Reduction in TMEM30A expression and increased PtS results in higher IDO-1 expression (converts IDO-1^{lo} to IDO-1^{hi})

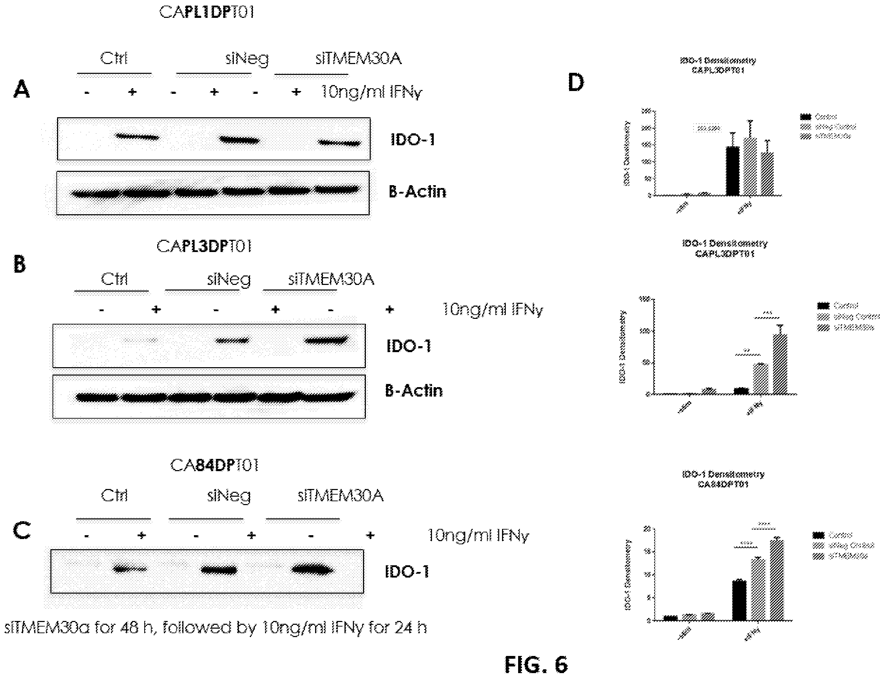

Three independent donor pools of ORBCEL-C^{™} (CAPL1DP **(****Fig. 6A****),** CAPL3DP **(****Fig. 6B****), C** CA84DP **(****Fig. 6C****))** were transfected with siRNA directed at TMEM30A or negative siRNA. Human IDO protein was determined by western blotting analysis. MSCs were cultured for 24 h, transfected with siTMEM30A/siNeg Ctrl for 48 h followed by stimulation for 24 h and harvested before total cell lysates were assayed for human IDO. Unstimulated MSCs served as a negative control; human MSCs stimulated with the inflammatory cytokine IFNγ (10 ng/mL) served as positive control. As shown in **Fig. 6D** (densitometry analysis of western blots shown in **Figs. 6A-C****),** expression of IDO-1 was significantly increased in CAPL3DP and CA84DP cells upon treatment with siRNA directed at TMEM30A.

### Example 18: TMEM30A knowndown (KD) Switches IDO-1^{lo} donor's M2 monocytes induction from Low to High

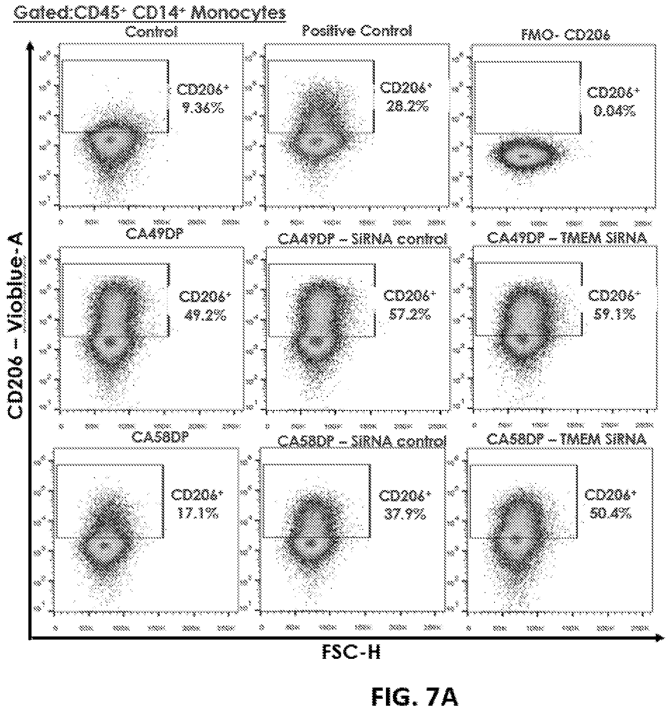

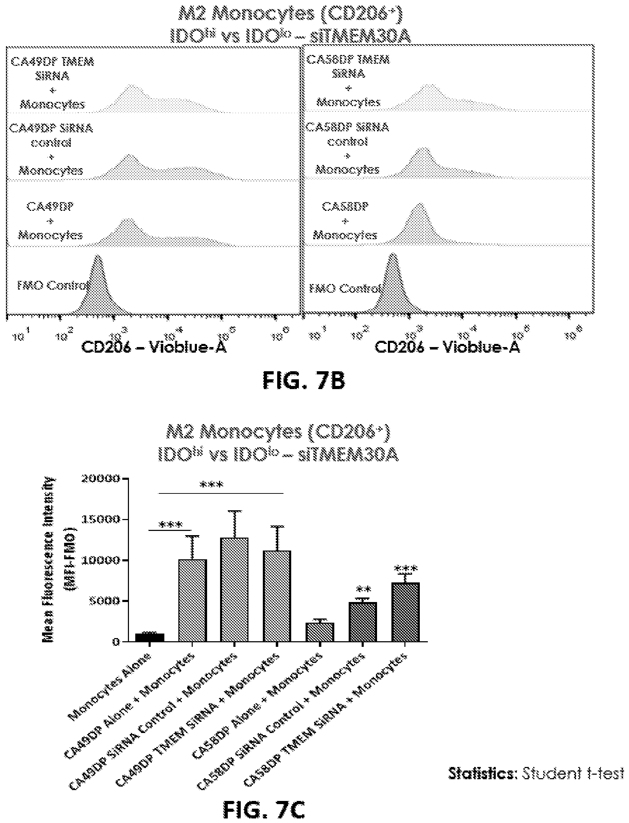

MSCs (ORBCEL-C) either TMEM30A siRNA knockdown MSCs or controls siRNA MSCs were cultured with freshly MACS isolated CD14⁺ monocytes for 24hrs. 24hrs post co-culture, cells were labelled & gated for CD45⁺ CD14⁺ CD206⁺ M2 monocytes. **Fig. 7A** shows FACS analysis of cell population in each condition: (Conditions - 1) Monocytes alone, 2) Monocytes + IDO-1^{hi} HUC49 DP (ORBCEL-C), 3) Monocytes + IDO-1lo HUC58 DP. 4) Monocytes + IDO-1^{hi} HUC49 DP SiRNA control, 5) Monocytes + IDO-1^{hi} HUC49 DP TMEM SiRNA, 6) Monocytes + IDO-1^{lo} HUC58 siRNA control & 7) Monocytes + IDO-1^{lo} HUC58 TMEM SiRNA). **Fig. 7B** shows a histogram depicting the expression of CD206 on cells gated for monocyte population (CD45⁺CD14⁺) from different culture conditions. **Fig. 7C** shows a representative bar graph showing Mean Fluorescence intensity (MFI) of CD206 expression on monocytes between different culture conditions (n=1). Statistics: Students T-test. As shown, TMEM30A knockdown switches IDO-1^{lo} donor's M2 monocytes induction from Low to High (increased CD206+ cells) while TMEM30A knockdown does not significantly affect the IDO-1^{high} donor's M2 monocytes induction.

### Example 19: Induction of regulatory monocytes (CD163⁺ CD206⁺) by IDO-1^{hi} DP is via an active cell-cell interaction process

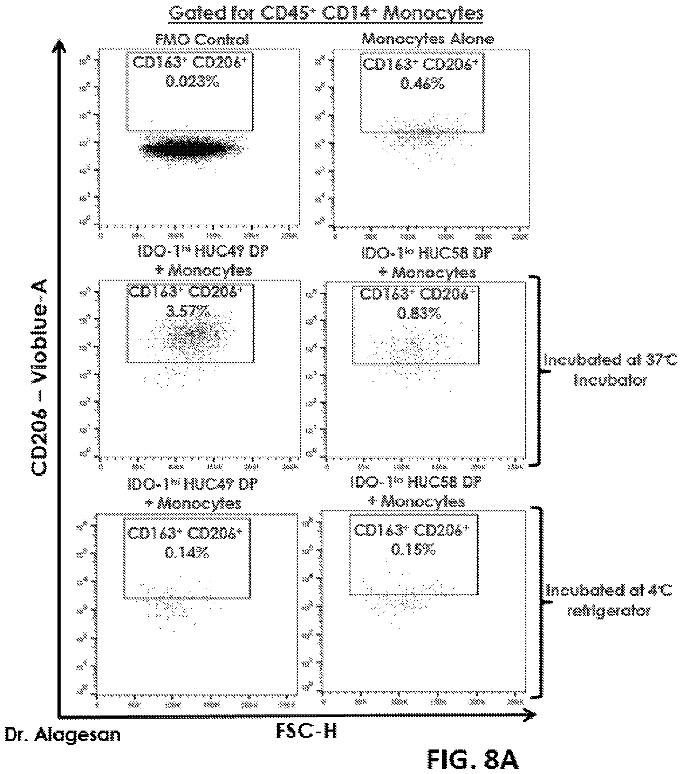

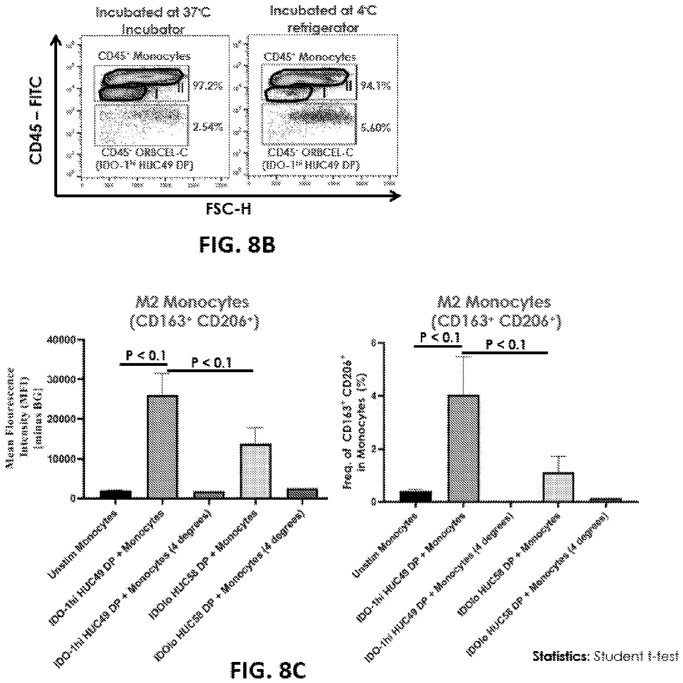

MSCs (ORBCEL-C) were cultured with freshly MACS isolated CD14⁺ monocytes for 24hrs either at 37°C incubator or at 4°C refrigerator. 24hrs post co-culture, cells were labelled & gated for CD45⁺ CD14⁺ CD163⁺ CD206⁺ M2 monocytes. **Fig. 8A** shows a FACS analysis of cell population in each condition: Conditions - 1) Monocytes alone, 2) Monocytes + IDO-1^{hi} HUC49 DP (ORBCEL-C) (37°C or 4°C) & 3) Monocytes + IDO-1^{lo} HUC58 DP (ORBCEL-C) (37°C or 4°C). **Fig 8B** shows flow cytometry dot plots showing two populations of CD45⁺ monocytes (I & II) modulated by MSCs (ORBCEL-C) and CD45⁻ IDO-1^{hi} HUC49 DP (ORBCEL-C) cultured either at 37°C or 4°C. Fig 8C shows Representative bar graph showing Mean Fluorescence intensity (MFI) of CD163⁺ CD206⁺ expression on monocytes & percentage of CD163⁺ CD206⁺ monocytes between different conditions (n=1). Statistics: Students T-test.

### Example 20: IDO-1^{hi} donor primed monocytes are capable of inducing regulatory T-cells (T-regs)

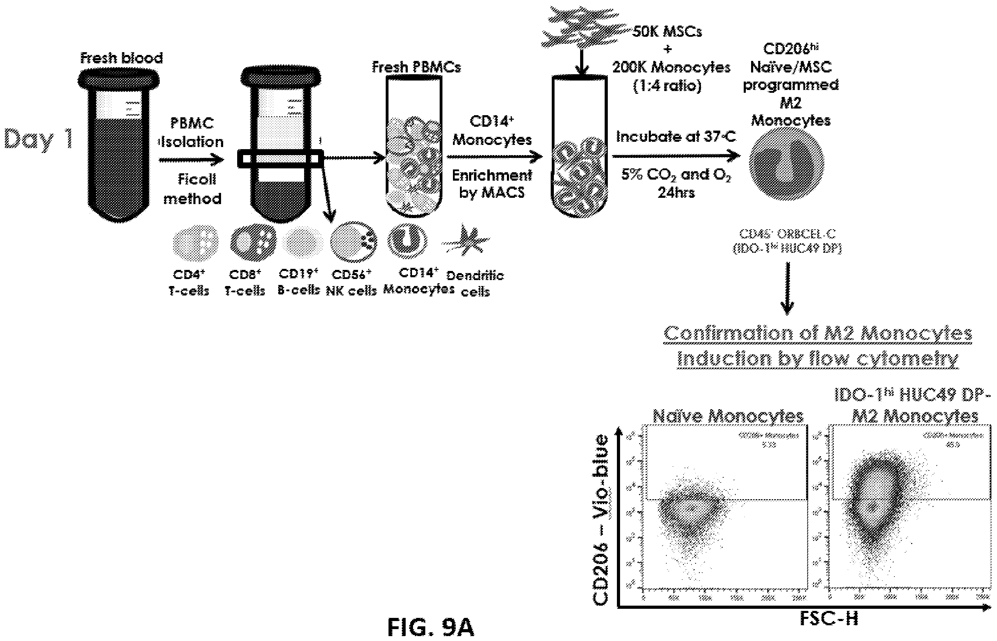

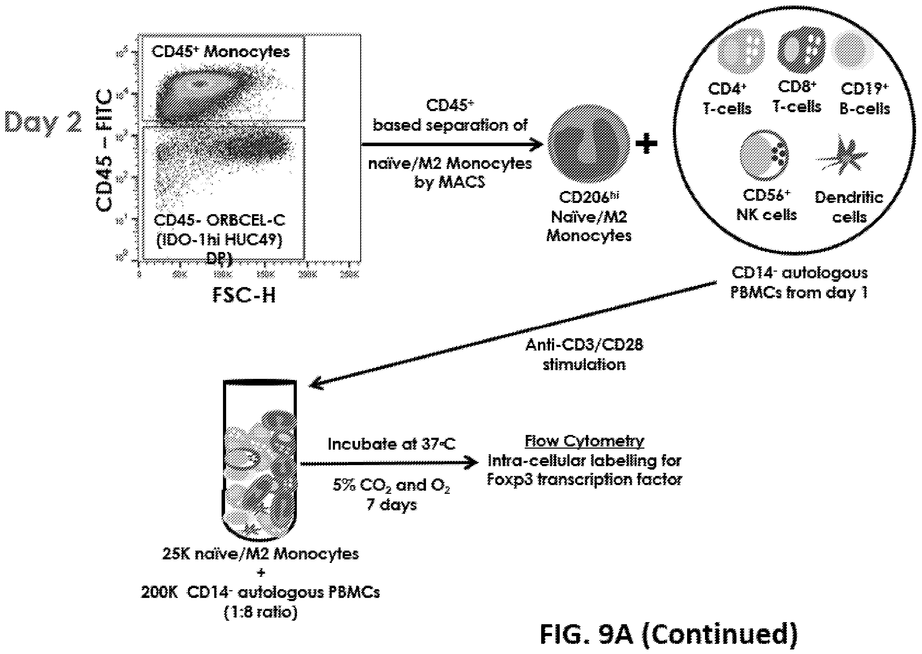

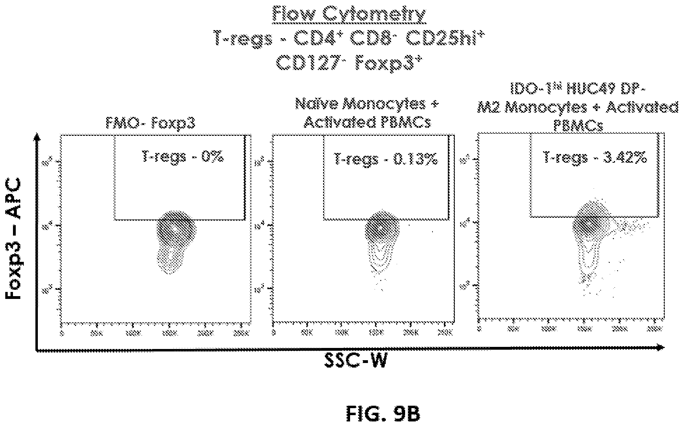

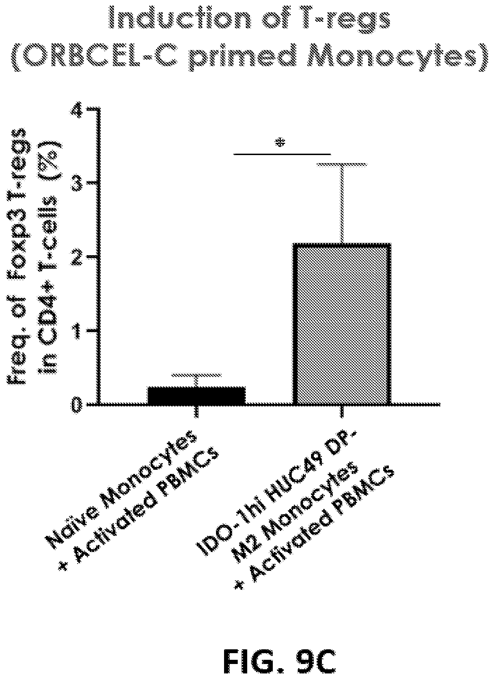

Monocytes and MSCs were co-cultured for 24 hrs. Following 24hrs co-culture of MSC, monocytes were purified by CD45⁺ MACS separation and cultured for 7 days with autologous CD3 CD28 activated PBMCs. **Fig. 9A** shows a schematic diagram explaining the co-culture of MSC primed monocytes: autologous PBMCs for T-reg assay. Regulatory T-cells were analysed by intra-cellular labelling of Foxp3 and gated for CD4⁺ CD8⁻ CD25hi⁺CD127⁻ Foxp3⁺ regulatory T-cells. **Fig. 9B** shows flow cytometry analysis of CD4⁺ CD8⁻ CD25hi⁺ CD127⁻ Foxp3⁺ regulatory T-cell population in various conditions: Conditions - 1) Naive Monocytes + Activated PBMCs, and 2) IDO-1^{hi} HUC49 DP - M2 Monocytes + Activated PBMCs. Fig. 9C shows a representative bar graph showing percentage of regulatory T-cells in CD4⁺ T-cells from different culture conditions (n=1). Statistics: Students T-test. As shown, the frequency of Foxp3⁺ regulatory T-cells among the CD4+ T cells was significantly increased when the monocytes were primed with ORBCEL-C.

## Claims

1. A population of SDC2+ stromal stem cells treated with a CDC50A inhibitory nucleic acid for use in inducing M2 phenotype polarization of monocytes and macrophages in a patient in need thereof.

2. The population of SDC2+ stromal stem cells for use according to claim 1, wherein the CDC50A inhibitory nucleic acid is a CDC50A shRNA or siRNA .

3. The population of SDC2+ stromal stem cells for use according to any one of claim 1 or claim 2, wherein the inhibitory nucleic acid is expressed by a virus.

4. The population of SDC2+ stromal stem cells for use according to any one of claims 1 to 3, wherein the population of SDC2+ stromal stem cells has CDC50A expression level reduced by at least 30% compared with an untreated population of SDC2+ stromal stem cells.

5. The population of SDC2+ stromal stem cells for use according to any one of claims 1 to 4, wherein the population of SDC2+ stromal stem cells has CDC50A activity reduced by at least 30% compared with an untreated population of SDC2+ stromal stem cells.

6. The population of SDC2+ stromal stem cells for use according to any one of claims 1 to 5, wherein the population of SDC2+ stromal stem cells has increased cell surface phosphatidyl serine expression compared with an untreated population of SDC2+ stromal stem cells.

7. The population of SDC2+ stromal stem cells for use according to any one of claims 1 to 6, wherein the population of SDC2+ stromal stem cells has increased itaconate in the cells compared with an untreated population of SDC2+ stromal stem cells.

8. The population of SDC2+ stromal stem cells for use according to any one of claims 1 to 7, wherein the SDC2+ stromal stem cells are isolated from umbilical cord or bone marrow.

9. The population of SDC2+ stromal stem cells for use according to any one of claims 1 to 8, wherein the SDC2+ stromal stem cells are human cells.

10. The population of SDC2+ stromal stem cells for use according to any one of claims 1 to 9, wherein the population is formulated in a physiologically acceptable buffer or excipient.

11. A method of preparing a population of stromal stem cells, the method comprising obtaining a population of mammalian cells; treating the population of mammalian cells with a CDC50A inhibitory nucleic acid; and isolating SDC2+ cells from the treated population of mammalian cells.

## Patentansprüche

1. Population von SDC2+ Stromastammzellen, die mit einer CDC50A-inhibitorischen Nukleinsäure behandelt sind, zur Verwendung beim Induzieren der M2-Phänotyppolarisation von Monozyten und Makrophagen bei einem Patienten, der dies benötigt.

2. Population von SDC2+ Stromastammzellen zur Verwendung nach Anspruch 1, wobei die CDC50A-inhibitorische Nukleinsäure eine CDC50A-shRNA oder -siRNA ist.

3. Population von SDC2+ Stromastammzellen zur Verwendung nach einem von Anspruch 1 oder Anspruch 2, wobei die inhibitorische Nukleinsäure durch ein Virus exprimiert ist.

4. Population von SDC2+ Stromastammzellen zur Verwendung nach einem der Ansprüche 1 bis 3, wobei die Population von SDC2+ Stromastammzellen ein um mindestens 30 % reduziertes CDC50A-Expressionsniveau im Vergleich zu einer unbehandelten Population von SDC2+ Stromastammzellen aufweist.

5. Population von SDC2+ Stromastammzellen zur Verwendung nach einem der Ansprüche 1 bis 4, wobei die Population von SDC2+ Stromastammzellen eine um mindestens 30 % reduzierte CDC50A-Aktivität im Vergleich zu einer unbehandelten Population von SDC2+ Stromastammzellen aufweist.

6. Population von SDC2+ Stromastammzellen zur Verwendung nach einem der Ansprüche 1 bis 5, wobei die Population von SDC2+ Stromastammzellen eine erhöhte Expression von Phosphatidylserin an der Zelloberfläche im Vergleich zu einer unbehandelten Population von SDC2+ Stromastammzellen aufweist.

7. Population von SDC2+ Stromastammzellen zur Verwendung nach einem der Ansprüche 1 bis 6, wobei die Population von SDC2+ Stromastammzellen erhöhtes Itaconat in den Zellen im Vergleich zu einer unbehandelten Population von SDC2+ Stromastammzellen aufweist.

8. Population von SDC2+ Stromastammzellen zur Verwendung nach einem der Ansprüche 1 bis 7, wobei die SDC2+ Stromastammzellen aus Nabelschnur oder Knochenmark isoliert sind.

9. Population von SDC2+ Stromastammzellen zur Verwendung nach einem der Ansprüche 1 bis 8, wobei die SDC2+ Stromastammzellen menschliche Zellen sind.

10. Population von SDC2+ Stromastammzellen zur Verwendung nach einem der Ansprüche 1 bis 9, wobei die Population in einem physiologisch unbedenklichen Puffer oder Hilfsstoff formuliert ist.

11. Verfahren zum Herstellen einer Population von Stromastammzellen, wobei das Verfahren Erlangen einer Population von Säugetierzellen; Behandeln der Population von Säugetierzellen mit einer CDC50A-inhibitorischen Nukleinsäure; und Isolieren von SDC2+ Zellen aus der behandelten Population von Säugetierzellen umfasst.

## Revendications

1. Population de cellules souches stromales SDC2+ traitées par un acide nucléique inhibiteur de CDC50A destinée à être utilisée dans l'induction de la polarisation du phénotype M2 de monocytes et de macrophages chez un patient en ayant besoin.

2. Population de cellules souches stromales SDC2+ destinée à être utilisée selon la revendication 1, dans laquelle l'acide nucléique inhibiteur de CDC50A est un ARNsh ou ARNsi CDC50A.

3. Population de cellules souches stromales SDC2+ destinée à être utilisée selon l'une quelconque des revendications 1 ou 2, dans laquelle l'acide nucléique inhibiteur est exprimé par un virus.

4. Population de cellules souches stromales SDC2+ destinée à être utilisée selon l'une quelconque des revendications 1 à 3, dans laquelle la population de cellules souches stromales SDC2+ comporte un niveau d'expression de CDC50A réduit d'au moins 30 % par rapport à une population non traitée de cellules souches stromales SDC2+.

5. Population de cellules souches stromales SDC2+ destinée à être utilisée selon l'une quelconque des revendications 1 à 4, dans laquelle la population de cellules souches stromales SDC2+ comporte une activité CDC50A réduite d'au moins 30 % par rapport à une population non traitée de cellules souches stromales SDC2+.

6. Population de cellules souches stromales SDC2+ destinée à être utilisée selon l'une quelconque des revendications 1 à 5, dans laquelle la population de cellules souches stromales SDC2+ comporte une expression accrue de phosphatidylsérine à la surface cellulaire par rapport à une population non traitée de cellules souches stromales SDC2+.

7. Population de cellules souches stromales SDC2+ destinée à être utilisée selon l'une quelconque des revendications 1 à 6, dans laquelle la population de cellules souches stromales SDC2+ comporte une teneur en itaconate accrue dans les cellules par rapport à une population non traitée de cellules souches stromales SDC2+.

8. Population de cellules souches stromales SDC2+ destinée à être utilisée selon l'une quelconque des revendications 1 à 7, dans laquelle les cellules souches stromales SDC2+ sont isolées du cordon ombilical ou de la moelle osseuse.

9. Population de cellules souches stromales SDC2+ destinée à être utilisée selon l'une quelconque des revendications 1 à 8, dans laquelle les cellules souches stromales SDC2+ sont des cellules humaines.

10. Population de cellules souches stromales SDC2+ destinée à être utilisée selon l'une quelconque des revendications 1 à 9, dans laquelle la population est formulée dans un tampon ou un excipient physiologiquement acceptable.

11. Procédé de préparation d'une population de cellules souches stromales, le procédé comprenant l'obtention d'une population de cellules de mammifère ; le traitement de la population de cellules de mammifère par un acide nucléique inhibiteur de CDC50A ; et l'isolement de cellules SDC2+ de la population traitée de cellules de mammifère.
